(19)

Europäisches Patentamt

European Patent Office

Office européen des brevets

(11) **EP 4 645 317 A1**

(12) **EUROPEAN PATENT APPLICATION**

(43) Date of publication:
**05.11.2025 Bulletin 2025/45**

(21) Application number: **25173115.4**

(22) Date of filing: **29.04.2025**

(51) International Patent Classification (IPC):
**G16B 20/20** (2019.01)  **G16B 30/20** (2019.01)
**G16B 40/20** (2019.01)

(52) Cooperative Patent Classification (CPC):
**G16B 40/20; G16B 20/20; G16B 30/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **30.04.2024  CN 202410540991**

(71) Applicant: **GeneMind Biosciences Company
Limited
Shenzhen City, 518023 Guangdong (CN)**

(72) Inventor: **CHEN, Weiyue
Shenzhen City, 518023 (CN)**

(74) Representative: **Schiweck Weinzierl Koch
Patentanwälte Partnerschaft mbB
Ganghoferstraße 68 B
80339 München (DE)**

(54) **BASE CALLING METHOD AND APPARATUS, DEVICE AND STORAGE MEDIUM**

(57)    Disclosed are a method and an apparatus for base calling, a device, and a storage medium. The method includes: acquiring a first mapping relationship between correct/incorrect classification information of an initial base calling result of a sequencing cycle and first sequencing information of the initial base calling result of a sequencing cycle, where the first sequencing information includes first initial base calling information based on a designated sequencing cycle; and determining the correct/incorrect classification information of the initial base calling result of a sequencing cycle to be processed based on the first mapping relationship and the first sequencing information of the sequencing cycle to be processed. The method for base calling according to the method reduces the impact of a contextual sequence on the base calling and improves the accuracy of the base calling.

Acquiring a mapping relationship between the standard base of a sequencing cycle and first sequencing information, where the first sequencing information includes first initial base calling information based on the designated sequencing cycle, as well as first initial base calling information based on a consecutive sequencing cycles, and the a consecutive sequencing cycles include the designated sequencing cycle — S110

Determining a base calling result of the sequencing cycle to be processed based on the mapping relationship and the first sequencing information of the sequencing cycle to be processed — S120

FIG. 1

EP 4 645 317 A1

**Description**

TECHNICAL FIELD

The present disclosure relates to the technical field of biological information processing, and in particular to a method and an apparatus for base calling, a device, and a storage medium.

BACKGROUND

DNA sequencing is one of the most fundamental technologies in modern life sciences. The high-quality sequencing data provided by DNA sequencing technologies depends on accurate base calling during the sequencing process. However, all sequencing platforms have a certain base calling error rate, and these errors may affect downstream bioinformatics analysis results, and consequently, the accuracy of corresponding research results. Therefore, for any sequencing platform, reducing the error rate is the key to improving the sequencing quality. Accordingly, how to improve the accuracy of base calling has become an important research focus.

SUMMARY

The present disclosure provides a method and an apparatus for base calling, a device, and a storage medium for improving the accuracy of base calling.

According to an aspect of the present disclosure, provided is a method for base calling, including:

acquiring a first mapping relationship between correct/incorrect classification information of an initial base calling result of a sequencing cycle and first sequencing information of the initial base calling result of a sequencing cycle, where the first sequencing information includes first initial base calling information based on a target sequencing cycle; and determining the correct/incorrect classification information of the initial base calling result of a sequencing cycle to be processed based on the first mapping relationship and the first sequencing information of the sequencing cycle to be processed.

According to another aspect of the present disclosure, provided is an apparatus for base calling, including:

a first mapping relationship acquisition module, configured to acquire a first mapping relationship between correct/incorrect classification information of an initial base calling result of a sequencing cycle and first sequencing information of the initial base calling result of a sequencing cycle, where the first sequencing information includes first initial base calling information based on a target sequencing cycle,

the first initial base calling information includes initial base calling information of *a* consecutive sequencing cycles, and the *a* consecutive sequencing cycles at least include the target sequencing cycle; and a correct/incorrect classification information determination module, configured to determine the correct/incorrect classification information of the initial base calling result of a sequencing cycle to be processed based on the first mapping relationship and the first sequencing information of the sequencing cycle to be processed.

According to another aspect of the present disclosure, provided is an electronic device, including: at least one processor; and

a memory in communication connection to the at least one processor, where the memory stores one or more computer programs run by the at least one processor, and the one or more computer programs, when run by the at least one processor, cause the at least one processor to perform the method according to any one of the embodiments of the present disclosure.

According to another aspect of the present disclosure, provided is a computer-readable storage medium. The computer-readable storage medium stores one or more computer instructions, and the one or more computer instructions, when executed by a processor, cause the processor to perform the method according to any one of the embodiments of the present disclosure.

According to the technical solutions of the embodiments of the present disclosure, a first mapping relationship between correct/incorrect classification information of an initial base calling result of a sequencing cycle and first sequencing information of the initial base calling result of a sequencing cycle is acquired, where the first sequencing information includes first initial base calling information based on a target sequencing cycle; and the correct/incorrect classification information of the initial base calling result of a sequencing cycle to be processed is determined based on the first mapping relationship and the first sequencing information of the sequencing cycle to be processed. Accordingly, by calibrating the base calling information of the sequencing cycle to be processed using the base calling information of *a* consecutive sequencing cycles, particularly by leveraging the characteristics of the target sequencing cycle and the contextual sequences thereof, the sequencing error rate can be significantly reduced and the impact of high-frequency errors can be effectively mitigated, thus improving the accuracy of base calling.

It should be understood that what is described in this section is not intended to identify key or critical features of the embodiments of the present disclosure, and it is also not intended to limit the scope of the present disclosure. Other features of the present disclosure will become apparent from the following description.

## BRIEF DESCRIPTION OF THE DRAWINGS

For clearer descriptions of the technical solutions according to the embodiments of the present disclosure, the drawings required to be used in the description of the embodiments are briefly introduced below. It is apparent that the drawings in the description below are only some embodiments of the present disclosure, and for those of ordinary skill in the art, other drawings may be obtained from the drawings without creative efforts.

FIG. 1 is a schematic flow chart of a method for base calling according to an embodiment of the present disclosure;
FIG. 2 is a schematic structural diagram of a base calling apparatus according to an embodiment of the present disclosure;
FIG. 3 is a schematic structural diagram of another base calling apparatus according to an embodiment of the present disclosure;

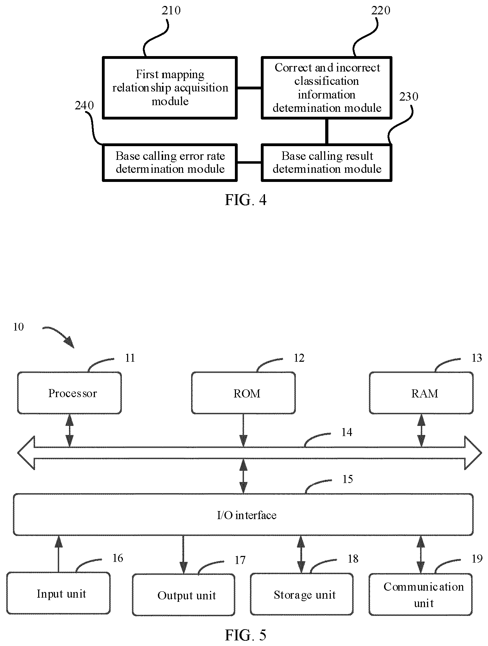

FIG. 4 is a schematic structural diagram of still another base calling apparatus according to an embodiment of the present disclosure; and
FIG. 5 is a schematic structural diagram of an electronic device according to an embodiment of the present disclosure.

## DETAILED DESCRIPTION

To enable those skilled in the art to better understand the solutions in the present disclosure, the technical solutions in the embodiments of the present disclosure will be clearly and completely described below with reference to the accompanying drawings in the embodiments of the present disclosure, and it is obvious that the described embodiments are only a part of the embodiments of the present disclosure but not all of them. Based on the embodiments of the present disclosure, all other embodiments obtained by those of ordinary skills in the art without creative effort shall fall within the protection scope of the present disclosure.

It should be noted that the terms "first", "second", etc. in the specification and claims of the present disclosure and the above accompanying drawings are used to distinguish similar objects, and do not have to be used to describe a specific order or sequence. It should be understood that the data so used is interchangeable under appropriate circumstances such that the embodiments of the present disclosure described herein are capable of implementation in other sequences than those illustrated or described herein.

The term "sequencing" may also be referred to as "nucleic acid sequencing" or "gene sequencing", that is, the three terms are used interchangeably, and refer to the determination of the type and order of bases in a nucleic acid sequence, including sequencing by synthesis (SBS) and/or sequencing by ligation (SBL), DNA sequencing and/or RNA sequencing, and long fragment sequencing and/or short fragment sequencing (the long fragment and short fragment are defined relatively; e.g., nucleic acid molecules longer than 1 Kb, 2 Kb, 5 Kb, or 10 Kb may be referred to as long fragments, and nucleic acid molecules shorter than 1 Kb or 800 bp may be referred to as short fragments).

Sequencing generally involves multiple cycles of sequencing to determine the order of multiple nucleotides/bases on the template: "one cycle of sequencing" (cycle), also referred to as a "sequencing cycle," may be defined as one base extension of four types of nucleotides/bases, and in other words, as the completion of the determination of the base type at any given position on a nucleic acid template. For sequencing platforms that achieve sequencing on the basis of polymerization reactions or ligation reactions, one cycle of sequencing includes a process of binding four types of nucleotides (including nucleotide analogs) to the corresponding nucleic acid template at a time and collecting corresponding signals generated by the four types of nucleotides (including nucleotide analogs) after binding. Generally, one cycle of sequencing may include one or more base extensions (repeats). For example, four types of nucleotides are sequentially added to the reaction system to perform base extensions and corresponding acquisition of reaction signals respectively, and in this case, one cycle of sequencing includes four base extensions; for another example, four types of nucleotides are added into the reaction system in any combinations (such as in pairs or in one-three combinations), base extensions and corresponding acquisition of reaction signals are performed for the two combinations respectively, and in this case, one cycle of sequencing includes two base extensions; for yet another example, four types of nucleotides are added simultaneously to the reaction system for base extension and reaction signal acquisition, and in this case, one cycle of sequencing includes one base extension.

Sequencing may be performed through sequencing platforms. According to the embodiments of the present application, available sequencing platforms include, but are not limited to, the Hiseq, Miseq, Nextseq, and Novaseq sequencing platforms of Illumina, the Ion Torrent platform of Thermo Fisher/Life Technologies, the BGISEQ and MGISEQ/DNBSEQ platforms of BGI, and single-molecule sequencing platforms.

In the description herein, A represents adenine and may also represent adenine nucleotide or an analog thereof; C represents cytosine and may also represent cytosine nucleotide or an analog thereof; G represents guanine and may also represent guanine nucleotide or an analog thereof; T represents thymine and may also represent thymine nucleotide or an analog thereof; U represents uracil and may also represent uracil nucleotide or an analog thereof. It should be understood that the representations of A, C, G, and T/U are consistent in the embodiments of the present disclosure. When one of them represents a base, the other three also represent bases. For example, when A represents adenine, correspondingly, C represents cytosine, G represents guanine, T represents thymine/U represents uracil. When one of them represents a nucleotide or an analog thereof, the other three also represent nucleotides or analogs thereof. For example, when A can represent adenine nucleotide or an analog thereof, correspondingly, C represents cytosine nucleotide or an analog thereof, G represents guanine nucleotide or an analog thereof, T represents thymine nucleotide or an analog thereof/U represents uracil or an analog thereof. "/" in T/U means "or", that is: "T/U" means "T or U".

In the description herein, unless otherwise specifically defined, based on image information, the terms "intensity" and pixel (pixel value) are used interchangeably, and the intensity or pixel may be a real or objective absolute value, or may be a relative value including various variations based on the real pixel value, such as an increased pixel value, a reduced pixel value, a proportion or relationship based on the pixel value. Generally, when comparison between a plurality of images or spots or positions in intensity/pixel is involved, the intensity/pixel of the images or spots or positions is the intensity/pixel after the same processing, such as objective pixel values or pixel values after the same transformation; and when comparison and analysis based on information of particular positions in one or more images are involved and the particular positions are determined, the images are preferably aligned and kept in the same coordinate system when determining these particular positions. In one embodiment, the "intensity" referred to in the embodiments of the present disclosure may be "fluorescence intensity".

The "spot" on an image, also referred to as "peak", "bright dot", or "light dot", refers to a position on an image where the signal is relatively strong, e.g., where the signal is stronger than the surrounding signals, appearing as a relatively bright speckle or dot on the image. A spot or its location occupies one or more pixels. The signal of spot/position may come from the target molecule or from non-target substance. Detection of "spots" includes detection of the optical signal from a target molecule, such as an extended base or base cluster.

The term "crosstalk", also referred to as "laser-crosstalk" or "spectra-crosstalk", refers to the phenomenon that the signal corresponding to one base diffuses into the signal of another base; for sequencing platforms that use fluorescent molecules labeled differently to identify different bases, it may be detected that the signal of one fluorescent molecule diffuses into another fluorescence channel in one cycle of sequencing if the emission spectra of two or more selected fluorescent molecules overlap.

The term "reaction asynchrony", also referred to as "phasing", "phase imbalance", "dephasing", or "phase diversity", refers to the phenomenon of asynchrony of reactions between nucleic acid molecules in a group, such as a cluster of nucleic acid molecules, in a chemical reaction, including phasing or sequence lag and prephasing or sequence lead, and it is, in a sequencing platform that uses fluorescent molecules labeled differently to identify different bases, shown as the phenomenon that the signal of the fluorescent molecule corresponding to the base at a specific position is not zero in more than one cycle of sequencing. In general, sequencing is performed using nucleotides that are labeled with fluorescent molecules and have a blocking group. The blocking group on a nucleotide may prevent other nucleotides from binding to the next position on the template, and is, for example, an azido group attached to the 3' position of the nucleotide's glycosyl, and either dropping of the blocking group or failing to remove the blocking group prior to the next base extension will result in phasing.

The term "channel" refers to four types of channels formed in different ways during the sequencing process for screening and distinguishing four types of bases derived from A, C, G, and T (or U). For example, the channel may refer to four types of fluorescence signal optical channels formed by using different excitation lights, different fluorescence filters, and the like in the sequencing process for screening and distinguishing the four fluorescent bases derived from A, C, G, and T (or U). In the practice of sequencing, images are obtained by taking pictures of the four different fluorescence channels. Ideally, each fluorescence channel only contains the signal of the fluorescent base type corresponding to the channel, but in practical cases, due to the influence of fluorescence crosstalk, the fluorescence signals of other bases may also be present in each channel besides the fluorescence signal of the corresponding fluorescent base.

The term "base calling error rate" refers to: the ratio of the number of incorrectly identified (confirmed by alignment with a standard reference genome) bases, denoted as $N_2$, to the total number of identified bases, denoted as $N_1$. The "base calling error rate" is represented by P, and $P = N_2/N_1$.

Moreover, the terms "comprise", "include" and "provided with" and any variations thereof are intended to cover non-exclusive inclusion. For example, a process, method, system, product, or device including a series of steps or units is not necessarily limited to the explicitly listed steps or units, but may include other steps or units that are not explicitly listed or are inherent in the process, method, product, or device.

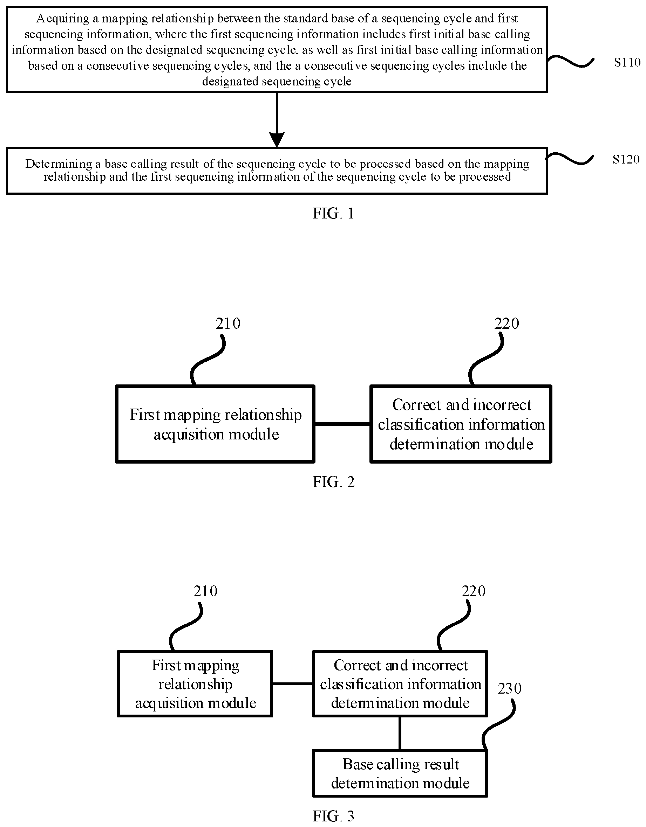

FIG. 1 is a schematic flow chart of a method for base calling according to an embodiment of the present disclosure. The embodiment may be applied to a case when a base calling result is determined according to sequencing information of a sequencing cycle, and the method may be performed by a base calling apparatus. The base calling apparatus may be implemented in the form of hardware and/or software, and the base calling apparatus may be configured in an electronic device.

As shown in FIG. 1, the method includes:
S110, acquiring a first mapping relationship between correct/incorrect classification information of an initial base calling result of a sequencing cycle and first sequencing information of the initial base calling result of a sequencing cycle.

In the embodiments of the present application, the first mapping relationship between the correct/incorrect classification information of the initial base calling result of the sequencing cycle and the first sequencing information refers to a relationship in which the correct/incorrect classification information of the initial base calling result of the sequencing cycle can be determined based on the first sequencing information of the sequencing cycle. The "correct/incorrect classification information" can also be referred to as "correct and incorrect classification information".

It should be understood that in the embodiments of the present application, the "sequencing cycle" in the "correct/incorrect classification information of the initial base calling result of a sequencing cycle" refers to a sequencing cycle to which the feature (first sequencing information) and the target (correct/incorrect classification information of initial base calling result) correspond. Without further explanation, a sequencing cycle generally refers to one sequencing cycle. In some embodiments, the sequencing cycle may also be understood as a target sequencing cycle or a designated sequencing cycle.

In the embodiments of the present application, the initial base calling result refers to an initial result obtained when performing base extension reactions in a sequencing cycle, where the sequencing platform identifies the base type of the targeted sequencing cycle (i.e., the target sequencing cycle) using existing base calling software. The output form of the initial result may be a base type, such as an A base, a T or U base, a G base, and a C base; or may be a probability score of each base type being detected or identified, such as a probability score of 94% for an A base, 2% for a T or U base, 2% for a G base, and 2% for a C base. It should be understood that in the conventional base calling software, as a general understanding, the base type of the sequencing cycle outputted from the base calling software may be determined by the probability of each base type, if not specified otherwise. Specifically, the base with the highest probability among the four types of bases is considered as the base type identified by the base calling software.

In the embodiments of the present application, the correct/incorrect classification information of the initial base calling result refers to information used to determine whether the initial base calling result is correctly classified or incorrectly classified. In some embodiments, the correct/incorrect classification information of the initial base calling result includes a correct classification and an incorrect classification of the identified base type. In some other embodiments, the correct/incorrect classification information of the initial base calling result includes probability scores for the correct classification and the incorrect classification of the accuracy of the identified base type. It should be understood that as a general understanding, the correct classification and the incorrect classification may be determined by the probability scores of the bases identified by the base calling software, if not specified otherwise.

In some embodiments, the correct/incorrect classification information may be correct and incorrect categories, including a correct category and an incorrect category. In some embodiments, the correct/incorrect category (also called "correct and incorrect category") may be a binary classification, i.e., a correct category and an incorrect category. In one implementation, if the initial base calling result of the base extension in the sequencing cycle matches the real base, the initial base calling of the sequencing cycle is considered correct and is classified under the correct category in the correct/incorrect classification information. Illustratively, if the initial base calling result of the base extension in the sequencing cycle is A, and the reagent base type is also A, the initial base calling of the sequencing cycle is considered correct. In this case, it is classified under the correct category in the correct/incorrect classification information. In another implementation, if the initial base calling result of the base extension of the sequencing cycle does not match the real base, the initial base calling of the sequencing cycle is considered incorrect and is classified as the incorrect category in the correct/incorrect classification information. Illustratively, if the initial base calling result of the base extension in the sequencing cycle is A and the real base is T, the initial base calling of the sequencing cycle is considered incorrect. In this case, it is classified under the incorrect category in the correct/incorrect classification information.

In some embodiments, the incorrect category may be further classified based on different types of errors, such that the correct/incorrect category is a multi-class classification with more than two categories. In one embodiment, the correct/incorrect category is a five-class classification, and the five-class classification includes the following five categories:

a correct category of the initial base calling result;
an incorrect category of A base;
an incorrect category of T or U base;
an incorrect category of G base; and
an incorrect category of C base.

The classification criterion for the incorrect category of A base is as follows: the corresponding real base of the sequencing cycle is A, but the initial base calling result of the base extension in the sequencing cycle is not A, and in this case, the correct/incorrect category of the initial base calling of the sequencing cycle is considered to fall into: the incorrect category of A base.

In some embodiments, the incorrect category of A base may be further classified based on the initial base calling result of the base extension in the sequencing cycle, for example, a category where A base is misidentified as T, a category where A base is misidentified as G, and a category where A base is misidentified as C.

Similarly, the classification criterion for the incorrect category of T or U base is as follows: the corresponding real base of the sequencing cycle is T, but the initial base calling result of the base extension in the sequencing cycle is not T, and in this case, the correct/incorrect category of the initial base calling of the sequencing cycle is considered to fall into: the incorrect category of T or U base. In some embodiments, the incorrect category of T or U base may be further classified based on the initial base calling result of the base extension in the sequencing cycle, for example, a category where T or U base is misidentified as A, a category where T or U base is misidentified as G, and a category where T or U base is misidentified as C.

The classification criterion for the incorrect category of G base is as follows: the corresponding real base of the sequencing cycle is G, but the initial base calling result of the base extension in the sequencing cycle is not G, and in this case, the correct/incorrect category of the initial base calling of the sequencing cycle is considered to fall into: the incorrect category of G base. In some embodiments, the incorrect category of G base may be further classified based on the initial base calling result of the base extension in the sequencing cycle, for example, a category where G base is misidentified as T, a category where G base is misidentified as A, and a category where G base is misidentified as C.

The classification criterion for the incorrect category of C base is as follows: the corresponding real base of the sequencing cycle is C, but the initial base calling result of the base extension in the sequencing cycle is not C, and in this case, the correct/incorrect category of the initial base calling of the sequencing cycle is considered to fall into: the incorrect category of C base. In some embodiments, the incorrect category of C base may be further classified based on the initial base calling result of the base extension in the sequencing cycle, for example, a category where C base is misidentified as A, a category where C base is misidentified as G, and a category where C base is misidentified as T.

In the embodiments of the present application, the first sequencing information includes the first initial base calling information of a sequencing cycle where base calling is required, i.e. a target sequencing cycle.

Considering that when base calling is performed in a target sequencing cycle, the identifiable features may be influenced by factors such as the contextual base types and the base extension reactions thereof, therefore, in the embodiment, when the mapping relationship between the correct/incorrect classification information of the initial base calling result of the target sequencing cycle and the first sequencing information is constructed, the corresponding relationship between the base calling information of the contextual sequences of the base of the target sequencing cycle and the standard base of the target sequencing cycle is considered, such that the influence factor of "contextual sequences" can be considered in the base calling. In addition, the errors related to the contextual sequences of the base to be identified in the sequencing are corrected based on the obtained error features related to the contextual sequences of the base to be identified, such that the sequencing error rate is reduced, and the impact of high-frequency errors in the specific contextual environments described above is mitigated, thus improving the accuracy of base calling ultimately. It should be understood that the term "contextual sequence" as referred to in the embodiments of the present application refers to, taking the base of the target sequencing cycle as the boundary, a base sequence obtained through the completion of base extension reactions prior to the target sequencing cycle, i.e., "upstream sequence", and a base sequence obtained through base extension reactions after the completion of base extension reactions in the target sequencing cycle, i.e., "downstream sequence".

In view of this, the first initial base calling information includes initial base calling information of $a$ consecutive sequencing cycles, and the $a$ consecutive sequencing cycles at least include the target sequencing cycle.

In the embodiments of the present application, the $a$ consecutive sequencing cycles include the target sequencing cycle and at least one of the following two scenarios:

> $n_1$ sequencing cycles where base extension reactions are performed prior to the target sequencing cycle, and
> $m_1$ sequencing cycles where base extension reactions are performed after the target sequencing cycle.

That is, the $a$ consecutive sequencing cycles include three implementations:
In the first implementation, the $a$ consecutive sequencing cycles include the target sequencing cycle, and the $n_1$ sequencing cycles where base extension reactions are performed prior to the target sequencing cycle, i.e., the previous $n_1$ sequencing cycles. In this case, $a = n_1 + 1$.

In the second implementation, the $a$ consecutive sequencing cycles include the target sequencing cycle, and the $m_1$ sequencing cycles where base extension reactions are performed after the target sequencing cycle, i.e., the later $m_1$ sequencing cycles. In this case, $a = m_1 + 1$.

In the third implementation, the $a$ consecutive sequencing cycles include the target sequencing cycle, the $n_1$ sequencing cycles where base extension reactions are performed prior to the target sequencing cycle, i.e., the previous $n_1$ sequencing cycles, and $m_1$ sequen-

cing cycles where base extension reactions are performed after the target sequencing cycle, i.e., the later $m_1$ sequencing cycles. In this case, $a = n_1 + m_1 + 1$, and a is a natural number greater than or equal to 2. This implementation can take into account the impact of contextual sequences on base calling in sequencing cycles, such that the accuracy of base calling can be improved to a greater extent.

The values of $m_1$ and $n_1$ satisfy the following conditions: $n_1$ is an integer greater than or equal to 0, and $m_1$ is an integer greater than or equal to 0. When $m_1$ and $n_1$ are simultaneously 0, the a consecutive sequencing cycles are the target sequencing cycle, i.e., the sequencing cycle requiring base calling. In this case, the first initial base calling information sequence only contains optical signals generated from the target sequencing cycles. Therefore, in the embodiments of the present application, $m_1$ and $n_1$ are not simultaneously 0, that is: at least one of $m_1$ and $n_1$ is not 0. Illustratively, at least one of $m_1$ and $n_1$ being not 0 includes the following cases: $m_1$ is 0 while $n_1$ is not 0; $m_1$ is not 0 while $n_1$ is 0; $m_1$ is not 0 and $n_1$ is not 0.

In some embodiments, $m_1$ and $n_1$ are each selected from natural numbers from 1 to 50. Illustratively, $m_1$ may be selected from 1, 2, 3, 4, 5, 8, 10, 12, 15, 18, 20, 22, 25, 28, 30, 32, 35, 38, 40, 42, 45, 48, 50, etc., and $n_1$ may be selected from 1, 2, 3, 4, 5, 8, 10, 12, 15, 18, 20, 22, 25, 28, 30, 32, 35, 38, 40, 42, 45, 48, 50, etc. It should be understood that the above values are merely examples, and the actual values of $m_1$ and $n_1$ are not limited thereto.

It should be understood that in the case that the a consecutive sequencing cycles include both the $n_1$ sequencing cycles where base extension reactions are performed prior to the target sequencing cycle, i.e., the previous $n_1$ sequencing cycles, and the $m_1$ sequencing cycles where base extension reactions are performed after the target sequencing cycle, i.e., the later $m_1$ sequencing cycles, the selection of $m_1$ and $n_1$ is not strictly required, and $m_1$ and $n_1$ may be the same or different.

In the embodiments of the present application, the first initial base calling information may be selected in different ways. For example, the first initial base calling information may be the initial base calling information sequence of a consecutive sequencing cycles, or the initial base calling information of each of a consecutive sequencing cycles, or the initial base calling information sequence formed by a part of consecutive sequencing cycles and the initial base calling information of each of the remaining sequencing cycles in a consecutive sequencing cycles.

In one implementation, the first initial base calling information includes the initial base calling information sequence of a consecutive sequencing cycles, i.e., the contextual sequences of the target sequencing cycle (including the target sequencing cycle). In this case, the first initial base calling information is a sequence composed of a consecutive pieces of base calling information. Illustratively, the initial base calling information sequence may be the initial base calling information sequence of $n_1 + 1$ consecutive sequencing cycles including the target sequencing cycle, or the initial base calling information sequence of $m_1 + 1$ consecutive sequencing cycles including the target sequencing cycle, or the initial base calling information sequence of $n_1 + m_1 + 1$ consecutive sequencing cycles including the target sequencing cycle. In this case, the initial base calling information sequence, as a whole, is associated with the correct/incorrect classification information of the initial base calling result of the sequencing cycle.

In another implementation, the first initial base calling information includes at least one of the initial base calling information sequence of the $n_1$ sequencing cycles and the initial base calling information sequence of the $m_1$ sequencing cycles, as well as the initial base calling information of the target sequencing cycle. In some embodiments, the first initial base calling information may be the initial base calling information sequence of the $n_1$ consecutive sequencing cycles, as well as the initial base calling information of the target sequencing cycle. In this case, the initial base calling information sequence of the $n_1$ consecutive sequencing cycles, as a whole, together with the initial base calling information of the target sequencing cycle, is associated with the correct/incorrect classification information of the initial base calling result of the sequencing cycle. In some embodiments, the first initial base calling information may be the initial base calling information sequence of the $m_1$ consecutive sequencing cycles, as well as the initial base calling information of the target sequencing cycle. In this case, the initial base calling information sequence of the $m_1$ consecutive sequencing cycles, as a whole, together with the initial base calling information of the target sequencing cycle, is associated with the correct/incorrect classification information of the initial base calling result of the sequencing cycle. In some embodiments, the first initial base calling information may be a set of the initial base calling information sequence of the $n_1$ consecutive sequencing cycles, the initial base calling information sequence of the $m_1$ consecutive sequencing cycles, and the initial base calling information of the target sequencing cycle. In this case, the initial base calling information sequence of the $n_1$ consecutive sequencing cycles, as a whole, and the initial base calling information sequence of the $m_1$ consecutive sequencing cycles, as a whole, together with the initial base calling information of the target sequencing cycle, are associated with the correct/incorrect classification information of the initial base calling result of the sequencing cycle.

In yet another implementation, the first initial base calling information includes the initial base calling information of each of the a consecutive sequencing cycles, i.e., a set of initial base calling information of each of the a consecutive sequencing cycles. In some embodiments, the first initial base calling information is a set formed by the initial base calling information of each of the $n_1$ consecutive sequencing cycles and the initial base calling information of the target sequencing cycle. In this case, the initial base calling information of each of the $n_1 + 1$ consecutive sequencing cycles is collectively associated with the correct/incorrect classification information of the initial base calling result of the sequencing cycle. In some embodiments, the first initial base calling information is a set formed by the initial base calling information of each of the $m_1$ consecutive sequencing cycles and the initial base calling information of the target sequencing cycle. In this case, the initial base calling information of each of the $m_1 + 1$ consecutive sequencing cycles is collectively associated with the correct/incorrect classification information of the initial base calling result of the sequencing cycle. In some embodiments, the first initial base calling information is a set formed by the initial base calling information of each of the $n_1$ consecutive sequencing cycles, the initial base calling information of each of the $m_1$ consecutive sequencing cycles, and the initial base calling information of the target sequencing cycle. In this case, the initial base calling information of each of the $n_1 + m_1 + 1$ consecutive sequencing cycles is collectively associated with the correct/incorrect classification information of the initial base calling result of the sequencing cycle.

In the embodiments of the present application, the first initial base calling information refers to initial information obtained when performing base calling in *a* consecutive sequencing cycles including the target sequencing cycle. The initial information may be the true or objective raw data outputted from base calling, or may be relative data derived from processing the raw data outputted from base calling, but not serving as the final base calling result.

In some embodiments, the first initial base calling information is optical signal intensities generated during base extension reactions in the *a* consecutive sequencing cycles including the target sequencing cycle. In some embodiments, the optical signal intensity includes optical signal intensities of a plurality of optical signal channels, where the plurality of optical signals respectively correspond to a plurality of base types involved in the base extension reaction. In this embodiment, there is a corresponding relationship between the optical signal and the base type and optical signal channel. In one embodiment, each base type corresponds to one optical signal channel, and the signals generated by such type of base in the optical signal channel are collected to obtain corresponding optical signals, such as a set of optical signals of a specific wavelength. Illustratively, based on the base type, the optical signal channels used for optical signal collection are four channels, including an A base channel, a C base channel, a G base channel, and a T/U base channel. In another embodiment, two or more base types share the same optical signal channel and their respective optical signals are acquired separately. Each type of signal in the optical signal channel is collected to obtain optical signals corresponding to the number of base types, such as optical signal sets with two or more types of specific wavelengths, where each optical signal set contains all the optical signals obtained at one type of specific wavelength. Illustratively, based on the base type, the optical signal channels used for optical signal collection are two channels, including an A/T/U base channel and a G/C base channel.

In some embodiments, the first initial base calling information includes the optical signal intensity of each optical signal channel. For example, in a four-channel system, the optical signal intensities from the A base channel, C base channel, G base channel, and T/U base channel are used as components of the first initial base calling information.

In some embodiments, the first initial base calling information is the highest optical signal intensity among the plurality of optical signal channels. For example, in a four-channel system, if the optical signal intensity in the A base channel is 0.9, and the optical signal intensities in the C base channel, the G base channel, and the T/U base channel are 0.2, 0.2, and 0.4, respectively, then the optical signal intensity in the A base channel is used as a component of the first initial base calling information.

In an embodiment where the base fluorescence signal serves as the base calling signal, the optical signal intensity is the fluorescence signal intensity. In this case, the first initial base calling information includes the fluorescence signal intensities of the fluorescence channels of the *a* consecutive sequencing cycles including the target sequencing cycle. Based on the base type, the fluorescence channels may include an A base fluorescence channel, a C base fluorescence channel, a G base fluorescence channel, and a T/U base fluorescence channel.

In the embodiments of the present application, the optical signal intensity includes any one of an initial optical signal intensity and a corrected optical signal intensity. The initial optical signal intensity refers to the optical signal intensity directly determined from an image, and the optical signal intensity can be obtained by extracting the intensity of optical signals from spots or valid spots in the image. The corrected optical signal intensity refers to an intensity obtained by correcting the initial optical signal intensity in a predetermined correction method. The predetermined correction method includes a background correction, a crosstalk correction, a reaction asynchrony correction, and the like. Additionally, a normalization process may also be regarded as one type of the correction method. Accordingly, the corrected intensity includes at least one of a background correction intensity, a crosstalk correction intensity, a reaction asynchrony correction intensity, and a normalization-processed intensity, but is not limited thereto. In some embodiments where the base fluorescence signal serves as the base calling signal, the optical signal intensity may be: the fluorescence intensity directly extracted from an optical image acquired by an optical imaging apparatus, i.e., the initial fluorescence intensity; or the relative fluorescence intensity obtained by processing the base image acquired by the optical imaging apparatus, such as performing background correction. Certainly, the optical signal intensity is not limited to the cases listed above, and may also be other data obtained by processing, for example, the fluorescence intensity in the above cases. In one embodiment, the first initial base calling information includes a sequence of corrected intensities obtained from the base extension reactions in *a* consecutive sequencing cycles including the target sequencing cycle. The scenarios of *a* consecutive sequencing cycles have been described above, and for brevity, the details are not repeated here.

Illustratively, the method for determining the corrected intensity includes the following steps: taking a four-channel system as an example, optical images acquired from the A base channel, C base channel, G base channel, and T/U base channel during the extension reactions in each sequencing cycle are collected respectively, and the raw optical signal intensity at each spot in the optical images is extracted; for each spot, the raw optical signal intensities from the A base channel, C base channel, G base channel, and T/U base channel are respectively taken as the initial intensities of the A base, C base, G base, and T/U base; and the corrected intensities of the A base, C base, G base, and T/U base are determined by performing at least one of a background correction, a crosstalk correction, a reaction asynchrony correction, and a normalization process for the initial intensities. In this embodiment, the corrected intensity is used as the source of the first initial base calling information, which eliminates interferences caused by factors such as background, crosstalk, and reaction asynchrony, thereby enhancing the purity of the sequencing signal. When the corrected intensity is used as a feature value and inputted into a corresponding machine learning and/or deep learning model for machine learning and/or deep learning, it is more conducive to improving the accuracy of model training.

In the embodiments of the present application, the first mapping relationship between the correct/incorrect classification information of the initial base calling result of a sequencing cycle and the first sequencing information may be a mapping mathematical model, a mapping neural network model, or the like. The first mapping relationship can be obtained by performing machine learning and/or deep learning using a predetermined machine learning and/or deep learning model, in which the sequencing information of the target sequencing cycle in a standard sample is used as a feature value and inputted, and the correct/incorrect classification information of the initial base calling result corresponding to the target sequencing cycle of the standard sample is used as a target value and inputted. That is, the mapping relationship between the correct/incorrect classification information of the initial base calling result of a sequencing cycle and the first sequencing information is obtained by training a pre-constructed Correct/Incorrect Base Calling Classification Model. The Correct/Incorrect Base Calling Classification Model is also call "model for correct and incorrect base calling result classification".

In some embodiments, the training of the Correct/Incorrect Base Calling Classification Model includes:

(11) acquiring a standard sample and a sequence of the standard sample;
(12) subjecting the standard sample to a base extension reaction, and determining second sequencing information of the target sequencing cycle and the initial base calling result of base extension in the target sequencing cycle, where the second sequencing information at least includes the first sequencing information;
(13) determining correct and incorrect categories of the initial base calling of the target sequencing cycle based on the initial base calling result of the base extension in the target sequencing cycle and a standard base;
(14) constructing a training sample by using the second sequencing information as a feature value and using the correct/incorrect category of the initial base calling sequence as a target value; and
(15) training the pre-constructed Correct/Incorrect Base Calling Classification Model based on the training sample to obtain a trained Correct/Incorrect Base Calling Classification Model.

In step (11) described above, the standard sample refers to a nucleic acid sample with a known base sequence. In the embodiments of the present application, the selection of the standard sample is not strictly required, and the standard sample may be a human reference gene sequence or a reference gene sequence of other species, as long as the sequence is known. In some embodiments, a sample with a larger genome is selected as the standard sample, and samples containing a plurality of different species may also be used together as the standard sample. By utilizing training data derived from samples with larger genomes or from genomes of multiple species, the training data can encompass a broader range of features, particularly the contextual sequence features required in the embodiments of the present application. This, in turn, enhances the generalization of the basecall model. It should be understood that "contextual sequence features" refer to sequencing information used for contextual base calling, such as the first sequencing information, the second sequencing information, and the like, as described above.

**In** the embodiments of the present application, the sequence of the standard sample can be directly obtained through the information provided by the standard sample, or determined by sequencing the standard sample and then aligning the obtained sequence with a reference genome. Illustratively, on a selected sequencing platform, the standard sample is sequenced to obtain the corresponding base sequence. The obtained base sequence is then aligned with the reference genome of the standard sample to determine the known sequence.

In step (12) described above, the standard sample is subjected to a base extension reaction to obtain the second sequencing information and the initial base calling result of the target sequencing cycle. In the embodiments of the present application, the initial base calling result of the target sequencing cycle refers to a result obtained when subjecting the standard sample to a base extension reaction, where the sequencing platform identifies the base type of the target sequencing cycle using existing base calling software. The result is a base type, such as an A base, a T or U base, a G base, and a C base.

In the embodiments of the present application, the second sequencing information refers to information associated with the base extension reaction in the target sequencing cycle of the standard sample and related to base calling. Theoretically, the broader the scope of information covered by the second sequencing information, the more features are encompassed in the training data, leading to better generalization of the basecall model and higher calibration accuracy of the basecall model. It should be understood that the second sequencing information at least includes the first sequencing information in the embodiments of the present application, that is, the first sequencing information is derived from the second sequencing information. In one embodiment, the first sequencing information is identical to the second sequencing information. In the embodiments of the present application, the second sequencing information at least includes: the first initial base calling information of $a$ consecutive sequencing cycles when subjecting the standard sample to a base extension reaction, where the $a$ consecutive sequencing cycles at least include the target sequencing cycle. The initial base calling information of the $a$ consecutive sequencing cycles may be referred to the descriptions provided above, and for brevity, the details are not repeated here.

In some embodiments, the first initial base calling information is a optical signal intensity generated during a base extension reaction. In some embodiments, the optical signal intensity includes optical signal intensities of a plurality of optical signal channels, where the plurality of optical signals respectively correspond to a plurality of base types involved in the base extension reaction. In some embodiments, the optical signal intensity is the highest optical signal intensity among the plurality of optical signal channels. In some embodiments, the optical signal intensity is a fluorescence signal intensity. In some embodiments, the fluorescence signal intensity includes any one of an initial fluorescence signal intensity and a corrected fluorescence signal intensity. In some embodiments, the correction includes at least one of a background correction, a crosstalk correction, a reaction asynchrony correction, and a normalization process. For brevity, the details are not repeated here.

In one implementation, the first initial base calling information includes the initial base calling information sequence of $a$ consecutive sequencing cycles, i.e., the contextual sequences of the target sequencing cycle (including the target sequencing cycle). In this case, the first initial base calling information is a sequence composed of a consecutive pieces of base calling information. Illustratively, the initial base calling information sequence may be the initial base calling information sequence of $n_1 + 1$ consecutive sequencing cycles including the target sequencing cycle, or the initial base calling information sequence of $m_1 + 1$ consecutive sequencing cycles including the target sequencing cycle, or the initial base calling information sequence of $n_1 + m_1 + 1$ consecutive sequencing cycles including the target sequencing cycle. In this case, the initial base calling information sequence, as a whole, is associated with the correct/incorrect classification information of the initial base calling result of the sequencing cycle.

In another implementation, the first initial base calling information includes at least one of the initial base calling information sequence of the $n_1$ sequencing cycles and the initial base calling information sequence of the $m_1$ sequencing cycles, as well as the initial base calling information of the target sequencing cycle. In some embodiments, the first initial base calling information may be the initial base calling information sequence of the $n_1$ consecutive sequencing cycles, as well as the initial base calling information of the target sequencing cycle. In this case, the initial base calling information sequence of the $n_1$ consecutive sequencing cycles, as a whole, together with the initial base calling information of the target sequencing cycle, is associated with the correct/incorrect classification information of the initial base calling result of the sequencing cycle. In some embodiments, the first initial base calling information may be the initial base calling information sequence of the $m_1$ consecutive sequencing cycles, as well as the initial base calling information of the target sequencing cycle. In this case, the initial base calling information sequence of the $m_1$ consecutive sequencing cycles, as a whole, together with the initial base calling information of the target sequencing cycle, is associated with the correct/incorrect classification information of the initial base calling result of the sequencing cycle. In some embodiments, the first initial base calling information may be a set of the initial base calling information sequence of the $n_1$ consecutive sequencing cycles, the initial base calling information sequence of the $m_1$ consecutive sequencing cycles, and the initial base calling information of the target sequencing cycle. In this case, the initial base calling information sequence of the $n_1$ consecutive sequencing cycles, as a whole, and the initial base calling information sequence of the $m_1$ consecutive sequencing cycles, as a whole, together with the initial base calling information of the target sequencing cycle, are associated with the correct/incorrect classification information of the initial base calling result of the sequencing cycle.

In yet another implementation, the first initial base calling information includes the initial base calling information of each of the $a$ consecutive sequencing cycles, i.e., a set of initial base calling information of each of the $a$ consecutive sequencing cycles. In some embodiments, the first initial base calling information is a set formed by the initial base calling information of each of the $n_1$ consecutive sequencing cycles and the initial base calling information of the target sequencing cycle. In this case, the initial base calling information of each of the $n_1 + 1$ consecutive sequencing cycles is collectively associated with the correct/incorrect classification information of the initial base calling result of the sequencing cycle. In some embodiments, the first initial base calling information is a set formed by the initial base calling information of each of the $m_1$ consecutive sequencing cycles and the initial base calling information of the target sequencing cycle. In this case, the initial base calling information of each of the $m_1 + 1$ consecutive sequencing cycles is collectively associated with the correct/incorrect classification information of the initial base calling result of the sequencing cycle. In some embodiments, the first initial base calling information is a set formed by the initial base calling information of each of the $n_1$ consecutive sequencing cycles, the initial base calling information of each of the $m_1$ consecutive sequencing cycles, and the initial base calling information of the target sequencing cycle. In this case, the initial base calling information of each of the $n_1 + m_1 + 1$ consecutive sequencing cycles is collectively associated with the correct/incorrect classification information of the initial base calling result of the sequencing cycle.

In step (13) described above, the correct and incorrect categories of the initial base calling of the target sequencing cycle are determined based on the alignment of the initial base calling result of the base extension in the target sequencing cycle with the corresponding standard base of the target sequencing cycle in the standard sample sequence.

In the embodiments of the present application, the correct and incorrect categories at least include a correct category and an incorrect category. In some embodiments, the correct/incorrect category may be a binary classification, i.e., a correct category and an incorrect category. In one implementation, if the initial base calling result of the base extension in the target sequencing cycle matches the corresponding standard base of the target sequencing cycle in the standard sample sequence, the initial base calling of the target sequencing cycle is considered correct. Illustratively, if the initial base calling result of the base extension in the target sequencing cycle is A, and the corresponding standard base of the target sequencing cycle in the standard sample sequence is also A, the initial base calling of the target sequencing cycle is considered correct. In another implementation, if the initial base calling result of the base extension in the target sequencing cycle does not match the corresponding standard base of the target sequencing cycle in the standard sample sequence, the initial base calling of the target sequencing cycle is considered incorrect. Illustratively, if the initial base calling result of the base extension in the target sequencing cycle is A, and the corresponding standard base of the target sequencing cycle in the standard sample sequence is T, the initial base calling of the target sequencing cycle is considered incorrect.

In some embodiments, the incorrect category may be further classified based on different types of errors, such that the correct/incorrect category is a multi-class classification with more than two categories. In one embodiment, the correct/incorrect category is a five-class classification, and the five-class classification includes the following five categories:

a correct category of the initial base calling result;
an incorrect category of A base;
an incorrect category of T or U base;
an incorrect category of G base; and
an incorrect category of C base.

The classification criterion for the incorrect category of A base is as follows: the corresponding standard base of the target sequencing cycle in the standard sample sequence is A, but the initial base calling result of the base extension in the target sequencing cycle is not A, and in this case, the correct/incorrect category of the initial base calling of the target sequencing cycle is considered to fall into: the incorrect category of A base. In some embodiments, the incorrect category of A base may be further classified based on the initial base calling result of the base extension in the target sequencing cycle, for example, a category where A base is misidentified as T, a category where A base is misidentified as G, and a category where A base is misidentified as C.

Similarly, the classification criterion for the incorrect category of T or U base is as follows: the corresponding standard base of the target sequencing cycle in the standard sample sequence is T, but the initial base calling result of the base extension in the target sequencing cycle is not T, and in this case, the correct/incorrect category of the initial base calling of the target sequencing cycle is considered to fall into: the incorrect category of T or U base. In some embodiments, the incorrect category of T or U base may be further classified based on the initial base calling result of the base extension in the target sequencing cycle, for example, a category where T or U base is misidentified as A, a category where T or U base is misidentified as G, and a category where T or U base is misidentified as C.

The classification criterion for the incorrect category of G base is as follows: the corresponding standard base of the target sequencing cycle in the standard sample sequence is G, but the initial base calling result of the base extension in the target sequencing cycle is not G, and in this case, the correct/incorrect category of the initial base calling of the target sequencing cycle is considered to fall into: the incorrect category of G base. In some embodiments, the incorrect category of G base may be further classified based on the initial base calling result of the base extension in the target sequencing cycle, for example, a category where G base is misidentified as T, a category where G base is misidentified as A, and a category where G base is misidentified as C.

The classification criterion for the incorrect category of C base is as follows: the corresponding standard base of the target sequencing cycle in the standard sample sequence is C, but the initial base calling result of the base extension in the target sequencing cycle is not C, and in this case, the correct/incorrect category of the initial base calling of the target sequencing cycle is considered to fall into: the incorrect category of C base. In some embodiments, the incorrect category of C base may be further classified based on the initial base calling result of the base extension in the target sequencing cycle, for example, a category where C base is misidentified as A, a category where C base is misidentified as G, and a category where C base is misidentified as T.

In steps (14) and (15) described above, a training sample is constructed by using the second sequencing information of the target sequencing cycle determined when subjecting the standard sample to a base extension reaction as a feature value and the correct/incorrect category of the initial base calling as a target value. The pre-constructed Correct/Incorrect Base Calling Classification Model is trained based on the training sample to acquire a mapping relationship between the correct/incorrect classification information of the initial base calling result of the sequencing cycle and the second sequencing information, thereby obtaining a trained Correct/Incorrect Base Calling Classification Model.

In one implementation, the first initial base calling information includes the initial base calling information sequence of $a$ consecutive sequencing cycles, i.e., the contextual sequences of the target sequencing cycle (including the target sequencing cycle). In this case, the first initial base calling information is a sequence composed of $a$ consecutive pieces of base calling information. When training the Correct/Incorrect Base Calling Classification Model, the initial base calling information sequence of $a$ consecutive sequencing cycles, as a whole, is inputted into a pre-constructed machine learning model/deep learning model as one feature for training.

In another implementation, the first initial base calling information includes at least one of the initial base calling information sequence of the $n_1$ sequencing cycles and the initial base calling information sequence of the $m_1$ sequencing cycles, as well as the initial base calling information of the target sequencing cycle.

In some embodiments, the first initial base calling information may be the initial base calling information sequence of the $n_1$ consecutive sequencing cycles, as well as the initial base calling information of the target sequencing cycle. When training the Correct/Incorrect Base Calling Classification Model, the initial base calling information sequence of the $n_1$ consecutive sequencing cycles, as a whole, together with the initial base calling information of the target sequencing cycle, is inputted into a pre-constructed machine learning model/deep learning model as two features for training.

In some embodiments, the first initial base calling information may be the initial base calling information sequence of the $m_1$ consecutive sequencing cycles, as well as the initial base calling information of the target sequencing cycle. When training the Correct/Incorrect Base Calling Classification Model, the initial base calling information sequence of the $m_1$ consecutive sequencing cycles, as a whole, together with the initial base calling information of the target sequencing cycle, is inputted into a pre-constructed machine learning model/deep learning model as two features for training.

In some embodiments, the first initial base calling information may be a set of the initial base calling information sequence of the $n_1$ consecutive sequencing cycles, the initial base calling information sequence of the $m_1$ consecutive sequencing cycles, and the initial base calling information of the target sequencing cycle. When training the Correct/Incorrect Base Calling Classification Model, the initial base calling information sequence of the $n_1$ consecutive sequencing cycles, as a whole, and the initial base calling information sequence of the $m_1$ consecutive sequencing cycles, as a whole, together with the initial base calling information of the target sequencing cycle, are inputted into a pre-constructed machine learning model/deep learning model as three features for training.

In yet another implementation, the first initial base calling information includes the initial base calling information of each of the $a$ consecutive sequencing cycles, i.e., a set of initial base calling information of each of the $a$ consecutive sequencing cycles.

In some embodiments, the first initial base calling information is a set formed by the initial base calling information of each of the $n_1$ consecutive sequencing cycles and the initial base calling information of the target sequencing cycle. When training the Correct/Incorrect Base Calling Classification Model, the initial base calling information of each of $n_1 + 1$ consecutive sequencing cycles is inputted into a pre-constructed machine learning model/deep learning model as one feature ($n_1 + 1$ features in total) for training.

In some embodiments, the first initial base calling information is a set formed by the initial base calling information of each of the $m_1$ consecutive sequencing cycles and the initial base calling information of the target sequencing cycle. When training the Correct/Incorrect Base Calling Classification Model, the initial base calling information of each of $m_1 + 1$ consecutive sequencing cycles is inputted into a pre-constructed machine learning model/deep learning model as one feature ($m_1 + 1$ features in total) for training.

In some embodiments, the first initial base calling information is a set formed by the initial base calling information of each of the $n_1$ consecutive sequencing cycles, the initial base calling information of each of the $m_1$ consecutive sequencing cycles, and the initial base calling information of the target sequencing cycle. In this case, when training the Correct/Incorrect Base Calling Classification Model, the initial base calling information of each of the $n_1 + m_1 + 1$ consecutive sequencing cycles is inputted into a pre-constructed machine learning model/deep learning model as one feature ($n_1 + m_1 + 1$ features in total) for training.

In the embodiments of the present application, the mapping relationship between the correct/incorrect category of the sequencing cycle and the second sequencing information may be determined by a mathematical model, a neural network, and the like, which is not limited herein. It is only required to acquire the second sequencing information of a known sample and the correct/incorrect category of the initial base calling of the known sample. In other words, the correct/incorrect category of the initial base calling of the known sample is determined based on the second sequencing information of the known sample.

In step S120, the correct/incorrect classification information of the initial base calling result of a sequencing cycle to be processed is determined based on the first mapping relationship and the first sequencing information of the sequencing cycle to be processed.

In this step, the sequencing cycle where the base extension reaction is completed is used as a sequencing cycle to be processed, and the correct/incorrect classification information of the initial base calling result of the sequencing cycle to be processed is determined based on the first sequencing information of the sequencing cycle to be processed and a pre-constructed first mapping relationship. In this case, the sequencing cycle to be processed is the sequencing cycle requiring base calling, and is the targeted sequencing cycle, which corresponds to the target sequencing cycle described above. The first sequencing information of the sequencing cycle to be processed includes the first initial base calling information of *a* consecutive sequencing cycles, and the *a* consecutive sequencing cycles include the sequencing cycle to be processed. For the *a* consecutive sequencing cycles and the first initial base calling information thereof, reference may be made to the descriptions provided above, and the details are not repeated here.

In one embodiment, determining the correct/incorrect classification information of the initial base calling result of a sequencing cycle to be processed based on the first mapping relationship and the first sequencing information of the sequencing cycle to be processed includes:

S121, acquiring the first sequencing information of the sequencing cycle to be processed; and

S 122, determining the correct/incorrect classification information of the initial base calling result of the sequencing cycle to be processed based on the first sequencing information and the first mapping relationship.

In step S121, acquiring the first sequencing information of the sequencing cycle to be processed may be understood as collecting the first sequencing information of the sequencing cycle to be processed. For the content covered by the first sequencing information, reference may be made to the descriptions provided above, and the details are not repeated here.

In step S122, the method involves acquiring the first mapping relationship between the standard base of a sequencing cycle and the first sequencing information by utilizing a pre-trained machine learning model and/or deep learning model. This model is trained based on the standard base of the sequencing cycle and the first sequencing information. Accordingly, when performing base balling in the sequencing cycle of a sample under test, the correct/incorrect classification information of the initial base calling result of the sequencing cycle to be processed is determined based on the first sequencing information of the sequencing cycle to be processed and the first mapping relationship. Specifically, the first sequencing information of the sequencing cycle to be processed is inputted into the first mapping relationship, and based on the correlation between the first sequencing information and the correct/incorrect classification information of the initial base calling result of the sequencing cycle in the first mapping relationship, the correct/incorrect classification information of the initial base calling result of the sequencing cycle to be processed is determined.

In some embodiments, the correct/incorrect classification information of the initial base calling result is probability scores of the correct and incorrect categories, or probability values of the correct and incorrect categories. That is, the percentage value corresponding to each category in the correct and incorrect categories.

In some embodiments, the method for base calling may further include:

S130, determining the base calling result of the sequencing cycle to be processed based on the correct/incorrect classification information of the initial base calling result.

As one implementation, the correctness of the initial base calling result is classified as a correct identification result and used as the base calling result of the sequencing cycle to be processed. As another implementation, when the correct/incorrect category is the five-class classification as described above or classification with more classes, the base calling result of the sequencing cycle to be processed can also be determined based on the specific incorrect category in the incorrect classification. Illustratively, when the correct and incorrect classification of the initial base calling result falls into the incorrect category of A base, the A base is used as the base calling result of the sequencing cycle to be processed; when the correct and incorrect classification of the initial base calling result falls into the incorrect category of T or U base, the T or U base is used as the base calling result of the sequencing cycle to be processed; when the correct and incorrect classification of the initial base calling result falls into the incorrect category of G base, the G base is used as the base calling result of the sequencing cycle to be processed; when the correct and incorrect classification of the initial base calling result falls into the incorrect category of C base, the C base is used as the base calling result of the sequencing cycle to be processed.

In some embodiments, the correct/incorrect classification information of the initial base calling result is probability scores of the correct and incorrect categories, or probability values of the correct and incorrect categories. That is, the percentage value corresponding to each category in the correct and incorrect categories.

In some embodiments, step S130 includes:

S 131, acquiring a second mapping relationship between the standard base of the sequencing cycle and third sequencing information; and

S132, determining the base calling result of the sequencing cycle to be processed based on the second mapping relationship and the third sequencing information of the sequencing cycle to be processed.

In step S131 described above, the second mapping relationship between the standard base of the sequencing cycle and the third sequencing information refers to a relationship in which the standard base or the real base of the sequencing cycle can be determined based on the third sequencing information of the sequencing cycle.

In the embodiments of the present application, the standard base of the sequencing cycle refers to the real base of the sequencing cycle. The real base of the sequencing cycle is known for the standard sample, and the real base of the sequencing cycle is unknown for the unknown sample under test. Therefore, the real base of the sequencing cycle is referred to as the standard base of the sequencing cycle herein.

In the embodiments of the present application, the third sequencing information of the sequencing cycle at least includes:

(a) the correct/incorrect classification information of the initial base calling result obtained in step S120.

The correct/incorrect classification information of the initial base calling result is as described above, and for brevity, the details are not repeated here.

In some embodiments, the correct/incorrect classification information of the initial base calling result is the correct and incorrect categories of a binary classification, including the correct category and the incorrect category of the initial base calling result. In some other embodiments, the correct/incorrect classification information of the initial base calling result is a multi-class classification with more than two categories. In one embodiment, the correct/incorrect category is a five-class classification, and the five-class classification includes the following five categories:

a correct category of the initial base calling result;
an incorrect category of A base;
an incorrect category of T or U base;
an incorrect category of G base; and
an incorrect category of C base.

For the details and explanations of the correct and incorrect categories of the binary classification and the five-class classification described above, reference may be made to the descriptions provided above.

In some embodiments, the correct/incorrect classification information of the initial base calling result is probability scores of the correct and incorrect categories, or probability values of the correct and incorrect categories. That is, the percentage value corresponding to each category in the correct and incorrect categories.

In addition, the third sequencing information further includes:

(b) second initial base calling information based on the target sequencing cycle.

In some embodiments, the second initial base calling information includes the initial base calling information of the target sequencing cycle. That is, the second initial base calling information refers to initial information obtained when performing base calling in the target sequencing cycle. The initial information may be the true or objective raw data outputted from base calling, or may be relative data derived from processing the raw data outputted from base calling, but not serving as the final base calling result.

In some embodiments, the second initial base calling information is a optical signal intensity generated during a base extension reaction in the target sequencing cycle. In some embodiments, the optical signal intensity includes optical signal intensities of a plurality of optical signal channels, where the plurality of optical signals respectively correspond to a plurality of base types involved in the base extension reaction. In this embodiment, there is a corresponding relationship between the optical signal and the base type and optical signal channel. In one embodiment, each base type corresponds to one optical signal channel, and the signals generated by such type of base in the optical signal channel are collected to obtain corresponding optical signals, such as a set of optical signals of a specific wavelength. Illustratively, based on the base type, the optical signal channels used for optical signal collection are four channels, including an A base channel, a C base channel, a G base channel, and a T/U base channel. In another embodiment, two or more base types share the same optical signal channel and their respective optical signals are acquired separately. Each type of signal in the optical signal channel is collected to obtain optical signals corresponding to the number of base types, such as optical signal sets with two or more types of specific wavelengths, where each optical signal set contains all the optical signals obtained at one type of specific wavelength. Illustratively, based on the base type, the optical signal channels used for optical signal collection are two channels, including an A/T/U base channel and a G/C base channel.

In some embodiments, the second initial base calling information includes the optical signal intensity of each optical signal channel. For example, in a four-channel system, the optical signal intensities from the A base channel, C base channel, G base channel, and T/U base channel are used as components of the second initial base calling information.

In some embodiments, the second initial base calling information is the highest optical signal intensity among the plurality of optical signal channels. For example, in a four-channel system, if the optical signal intensity in the A base channel is 0.9, and the optical signal intensities in the C base channel, the G base channel, and the T/U base channel are 0.2, 0.2, and 0.4, respectively, then the optical signal intensity in the A base channel is used as a component of the second initial base calling information.

In an embodiment where the base fluorescence signal serves as the base calling signal, the optical signal intensity is the fluorescence signal intensity. In this case, the second initial base calling information includes the fluorescence signal intensities of the fluorescence channels of the target sequencing cycle. Based on the base type, the fluorescence channels may include an A base fluorescence channel, a C base fluorescence channel, a G base fluorescence channel, and a T/U base fluorescence channel.

In the embodiments of the present application, the optical signal intensity includes any one of an initial optical signal intensity and a corrected optical signal intensity. The initial optical signal intensity refers to the optical signal intensity directly determined from an image, and the optical signal intensity can be obtained by extracting the intensity of optical signals from spots or valid spots in the image. The corrected optical signal intensity refers to an intensity obtained by correcting the initial optical signal intensity in a predetermined correction method. The predetermined correction method includes a background correction, a crosstalk correction, a reaction asynchrony correction, and the like. Additionally, a normalization process may also be regarded as one type of the correction method. Accordingly, the corrected intensity includes at least one of a background correction intensity, a crosstalk correction intensity, a reaction asynchrony correction intensity, and a normalization-processed intensity, but is not limited thereto. In some embodiments where the base fluorescence signal serves as the base calling signal, the optical signal intensity may be: the fluorescence intensity directly extracted from an optical image acquired by an optical imaging apparatus, i.e., the initial fluorescence intensity; or the relative fluorescence intensity obtained by processing the base image acquired by the optical imaging apparatus, such as performing background correction. Certainly, the optical signal intensity is not limited to the cases listed above, and may also be other data obtained by processing, for example, the fluorescence intensity in the above cases. In one embodiment, the second initial base calling information includes corrected intensities obtained from base extension reactions in the target sequencing cycle.

Illustratively, the method for determining the corrected intensity includes the following steps: taking a four-channel system as an example, optical images acquired from the A base channel, C base channel, G base channel, and T/U base channel during the extension reactions in each sequencing cycle are collected respectively, and the raw optical signal intensity at each spot in the optical images is extracted; for each spot, the raw optical signal intensities from the A base channel, C base channel, G base channel, and T/U base channel are respectively taken as the initial intensities of the A base, C base, G base, and T/U base; and the corrected intensities of the A base, C base, G base, and T/U base are determined by performing at least one of a background correction, a crosstalk correction, a reaction asynchrony correction, and a normalization process for the initial intensities. In this embodiment, the corrected intensity is used as the source of the first initial base calling information, which eliminates interferences caused by factors such as background, crosstalk, and reaction asynchrony, thereby enhancing the purity of the sequencing signal. When the corrected intensity is used as a feature value and inputted into a corresponding machine learning and/or deep learning model for machine learning and/or deep learning, it is more conducive to improving the accuracy of model training.

In some embodiments, the third sequencing information further includes at least one of the following information:

(c) an E value from the target sequencing cycle;
(d) a sequence determined based on the E value of each sequencing cycle in b consecutive sequencing cycles;
(e) optical signal intensities generated in each sequencing cycle in b consecutive sequencing cycles.

For (c) an E value from the target sequencing cycle, the E value is used to evaluate the quality of sequencing data, and is generally indicative of the accuracy of the sequencing data. Specifically, the E value is the ratio of the maximum value of optical signal intensities of the four base types in optical detection channels to the sum of the optical signal intensities of the four base types. Illustratively, in a sequencing method where fluorescence signals are collected through four channels, for a spot corresponding to a nucleic acid template, the E value is the ratio of the optical signal intensity of the channel with the strongest optical signal among the detected optical signal intensities of the four channels to the sum of the optical signal intensities of the four channels.

For (d), a sequence determined based on the E value of each sequencing cycle in b consecutive sequencing cycles refers to an E value sequence formed by arranging the E value of each sequencing cycle in order of the sequencing cycles in the b consecutive sequencing cycles. The E value is the ratio of the maximum value of optical signal intensities among four base types in optical detection channels to the sum of the optical signal intensities of the four base types. This sequence, as a whole, is associated with the standard base of the target sequencing cycle. It should be understood that in machine learning and/or deep learning, the sequence determined by the E value of each sequencing cycle in the b consecutive sequencing cycles is used as a feature for training the basecall model.

In some embodiments, the b consecutive sequencing cycles include the target sequencing cycle and at least one of the following two scenarios:

$n_2$ sequencing cycles where base extension reactions are performed prior to the target sequencing cycle, and
$m_2$ sequencing cycles where base extension reactions are performed after the target sequencing cycle.

That is, the b consecutive sequencing cycles include three implementations: in the first implementation, the b consecutive sequencing cycles include the target sequencing cycle, and the $n_2$ sequencing cycles where base extension reactions are performed prior to the target sequencing cycle, i.e., the previous $n_2$ sequencing cycles, and in this case, $b = n_2 + 1$; in the second implementation, the b consecutive sequencing cycles include the target sequencing cycle, and the $m_2$ sequencing cycles where base extension reactions are performed after the target sequencing cycle, i.e., the later $m_2$ sequencing cycles, and in this case, $b = m_2 + 1$; in the third implementation, the b consecutive sequencing cycles include the target sequencing cycle, the $n_2$ sequencing cycles where base extension reactions are performed prior to the target sequencing cycle, i.e., the previous $n_2$ sequencing cycles, and the $m_2$ sequencing cycles where base extension reactions are performed after the target sequencing cycle, i.e., the later $m_2$ sequencing cycles, and in this case, $b = n_2 + m_2 + 1$, and b is a natural number greater than or equal to 2.

The values of $m_2$ and $n_2$ satisfy the following conditions: $n_2$ is an integer greater than or equal to 0, and $m_2$ is an integer greater than or equal to 0. When $m_2$ and $n_2$ are simultaneously 0, the b consecutive sequencing cycles are the target sequencing cycle, i.e., the sequencing cycle requiring base calling, which is repeated with (b). Therefore, in the embodiments of the present application, $m_2$ and $n_2$ are not simultaneously 0, that is: at least one of $m_2$ and $n_2$ is not 0. Illustratively, at least one of $m_2$ and $n_2$ being not 0 includes the following cases: $m_2$ is 0 while $n_2$ is not 0; $m_2$ is not 0 while $n_2$ is 0; $m_2$ is not 0 and $n_2$ is not 0.

In some embodiments, $m_2$ and $n_2$ are each selected from natural numbers from 1 to 50. Illustratively, $m_2$ may be selected from 1, 2, 3, 4, 5, 8, 10, 12, 15, 18, 20, 22, 25, 28, 30, 32, 35, 38, 40, 42, 45, 48, 50, etc., and $n_2$ may be selected from 1, 2, 3, 4, 5, 8, 10, 12, 15, 18, 20, 22, 25, 28, 30, 32, 35, 38, 40, 42, 45, 48, 50, etc. It should be understood that the above values are merely examples, and the actual values of $m_2$ and $n_2$ are not limited thereto.

It should be understood that in the case that the b consecutive sequencing cycles include both the $n_2$ sequencing cycles where base extension reactions are performed prior to the target sequencing cycle, i.e., the previous $n_2$ sequencing cycles, and the $m_2$ sequencing cycles where base extension reactions are performed after the target sequencing cycle, i.e., the later $m_2$ sequencing cycles, the selection of $m_2$ and $n_2$ is not strictly required, and $m_2$ and $n_2$ may be the same or different.

In some embodiments, the third sequencing information includes both (c) and (d) described above, that is: the third sequencing information includes both the E value from the target sequencing cycle and the sequence determined based on the E value from each sequencing cycle in the b consecutive sequencing cycles.

For (e), optical signal intensities generated in each sequencing cycle in b consecutive sequencing cycles refer to optical signal intensities obtained from each of the b consecutive sequencing cycles during base calling. For the description of the b consecutive sequencing cycles and the optical signal intensity, reference may be made to the descriptions provided above, and the details are not repeated here.

In some embodiments, the optical signal intensities generated in each sequencing cycle in b consecutive sequencing cycles include the optical signal intensities generated in the target sequencing cycle, and the optical signal intensities generated in each of the previous $n_2$ sequencing cycles, and/or the optical signal intensities generated in each of the later $m_2$ sequencing cycles.

In the embodiments of the present application, the second mapping relationship between the standard base of the sequencing cycle and the third sequencing information may be a mapping mathematical model, a mapping neural network model, or the like. The second mapping relationship can be obtained by performing machine learning and/or deep learning using a predetermined machine learning and/or deep learning model, in which the third sequencing information of the target sequencing cycle in a standard sample is used as a feature value and inputted, and the standard base of the standard sample is used as a target value and inputted. That is, the mapping relationship between the standard base of a sequencing cycle and the third sequencing information is obtained by training a pre-constructed basecall model.

In some embodiments, the training of the basecall model includes:

(21) acquiring a standard sample and a sequence of the standard sample;
(22) subjecting the standard sample to a base extension reaction, and determining the third sequencing information of the sequencing cycle;
(23) constructing a training sample by using the third sequencing information of each sequencing cycle as a feature value and using the standard base corresponding to the sequencing cycle as a target value; and
(24) training the pre-constructed basecall model based on the training sample to obtain a trained basecall model.

In step (21) described above, the standard sample refers to a nucleic acid sample with a known base sequence. In the embodiments of the present application, the selection of the standard sample is not strictly required, and the standard sample may be a human reference gene sequence or a reference gene sequence of other species, as long as the sequence is known. In some embodiments, a sample with a larger genome is selected as the standard sample, and samples containing a plurality of different species may also be used together as the standard sample. By utilizing training data derived from samples with larger genomes or from genomes of multiple species, the training data can encompass a broader range of features, particularly the contextual sequence features required in the embodiments of the present application. This, in turn, enhances the generalization of the basecall model.

The sequence of the standard sample can be directly obtained through the information provided by the standard sample, or determined by sequencing the standard sample and then aligning the obtained sequence with a reference genome. Illustratively, on a selected sequencing platform, the standard sample is sequenced to obtain the corresponding base sequence. The obtained base sequence is then aligned with the reference genome of the standard sample to determine the known sequence.

In step (22) described above, the standard sample is subjected to a base extension reaction to acquire the third sequencing information of the sequencing cycle. In the embodiments of the present application, the third sequencing information is as described above, and the details are not repeated here.

In steps (23) and (24) described above, a training sample is constructed by using the third sequencing information of each sequencing cycle as a feature value and using the standard base corresponding to the sequencing cycle as a target value. The pre-constructed basecall model is trained based on the training sample to acquire the mapping relationship between the third sequencing information of the sequencing cycle and the standard base, thereby obtaining the trained basecall model.

It should be understood that when the third sequencing information contains sequence features in training the Correct/Incorrect Base Calling Classification Model, the sequence, as a whole, is inputted into a pre-constructed machine learning model/deep learning model as one feature for training.

In step S132 described above, the base calling result of the sequencing cycle to be processed can be determined based on the second mapping relationship and the third sequencing information of the sequencing cycle to be processed. In some embodiments, the output form of the base calling result may be a base type, such as an A base, a T or U base, a G base, and a C base; or may be a probability score of each base type being detected or identified, for example, a probability score of 94% for an A base, 2% for a T or U base, 2% for a G base, and 2% for a C base. It should be understood that in the conventional base calling software, as a general understanding, the base type of the sequencing cycle outputted from the base calling may be determined by the probability of each base type, if not specified otherwise. Specifically, the base with the highest probability among the four types of bases is considered as the base type obtained by base calling.

In one embodiment, in step S132, determining the initial base calling result of the sequencing cycle to be processed based on the second mapping relationship and the third sequencing information of the sequencing cycle to be processed includes:

acquiring the third sequencing information of the sequencing cycle to be processed; and
determining probabilities of four base types of the sequencing cycle to be processed based on the third sequencing information and the second mapping relationship, and taking the base type with the highest probability as the base calling result of the sequencing cycle to be processed.

Thus, the method for base calling provided in this embodiment involves two types of machine learning/deep learning models. The first type is a model for correct and incorrect base calling classification, which involves independent training and learning for the impact of sequence context on the correctness of sequencing in the target sequencing cycle and then incorporating the model results into the training of the basecall model. Specifically, a model for correct and incorrect base calling classification based on contextual sequence information is first established, followed by the establishment of a basecall model. This approach reduces the dimensionality of the feature space of the basecall model, thereby preventing the curse of dimensionality, avoiding the need for large-scale training data and computational power, lowering the risk of overfitting, and improving the stability and generalization of the model. Specifically, the model for correct and incorrect base calling classification requires a larger amount of training data, whereas the basecall model, although requiring relatively less training data, necessitates extracting a greater number of feature parameters for each training data entry. Although training the model for correct and incorrect base calling classification requires a larger amount of training data, the number of feature values is relatively small (e.g., the model for correct and incorrect base calling classification may have only one feature value, where the sequence context is merged as a single feature, or two feature values, where the upstream and downstream contexts are treated as separate features). As a result, the memory burden during storage and training is relatively low. The features required for training the basecall model include the fluorescence brightness of four channels corresponding to the current base (four features) and may include the current E value, the fluorescence brightness of the adjacent preceding and succeeding sequencing cycles (a total of eight features), and the like, resulting in a significant increase in the number of features. Compared to extracting the features required for both models together for training, the two models are trained independently in this embodiment, which does not require the use of identical training datasets or training data volumes for both models, nor does it necessitate a large number of training data entries. Consequently, this method offers advantages in terms of computational power, storage, training time, and model stability.

In one possible embodiment, the method for base calling further includes:

S133, determining a base calling error rate of the sequencing cycle to be processed based on the base calling result.

In this embodiment, the base calling result is the probability score of each base type being detected or identified, i.e., the base calling probability scores of the four base types. By integrating the probability score of each base type being detected or identified, the base calling error rate of the sequencing cycle to be processed is determined.

In some embodiments, in step S133, determining the base calling error rate of the sequencing cycle to be processed based on the base calling result includes:

S1331, acquiring a third mapping relationship between fourth sequencing information of the sequencing cycle and the base calling error rate; and

S1332, determining the base calling error rate of the sequencing cycle to be processed based on the third mapping relationship and the fourth sequencing information of the sequencing cycle to be processed.

In step S1331 described above, the third mapping relationship between the base calling error rate of the sequencing cycle and the fourth sequencing information refers to a relationship in which the base calling error rate of the sequencing cycle can be determined based on the fourth sequencing information of the sequencing cycle.

In the embodiments of the present application, the base calling error rate is used to characterize the accuracy of the base calling result of the sequencing cycle. In some embodiments, the base calling error rate can represent the error probabilities of the four base types, and can be characterized using a Q value, which is calculated as follows:

$$Q = -10 \times \log 10(P),$$

where Q represents the quality score and P represents the base calling error probability.

In the embodiments of the present application, the fourth sequencing information of the sequencing cycle is information associated with the features generated from the base extension reaction of the sequencing cycle. In some embodiments, the fourth sequencing information at least includes the base calling probability scores of four base types. Specifically, the fourth sequencing information includes the initial base calling result determined in step S132, and the initial base calling result is the probability score of each base type being detected or identified, i.e., the base calling probability scores of the four base types. In some embodiments, the fourth sequencing information further includes the correct/incorrect classification information of the initial base calling result obtained in step S120. In one implementation, the correct/incorrect category of the correct/incorrect classification information of the initial base calling result is a five-class classification, and the five-class classification includes the following five categories:

a correct category of the initial base calling result;
an incorrect category of A base;
an incorrect category of T or U base;
an incorrect category of G base; and
an incorrect category of C base.

For the details and explanations of the correct and incorrect categories of the five-class classification, reference may be made to the descriptions provided above, and for brevity, the details are not repeated here.

In the embodiments of the present application, the third mapping relationship between the fourth sequencing information of the sequencing cycle and the base calling error rate may be a mapping mathematical model, a mapping neural network model, or the like. The third mapping relationship can be obtained by performing machine learning and/or deep learning using a predetermined machine learning and/or deep learning model, in which the fourth sequencing information of the target sequencing cycle in a standard sample is used as a feature value and inputted, and the base calling error rate of the standard sample is used as a target value and inputted. That is, the mapping relationship between the base calling error rate of a sequencing cycle and the fourth sequencing information is obtained by training a pre-constructed base calling error rate model.

In some embodiments, the training of the base calling error rate model includes:

(31) acquiring a standard sample and a sequence of the standard sample;
(32) subjecting the standard sample to a base extension reaction, and determining the fourth sequencing information of the sequencing cycle;
(33) constructing a training sample by using the fourth sequencing information of each sequencing cycle as a feature value and using the base calling error rate corresponding to the sequencing cycle as a target value; and
(34) training the pre-constructed base calling error rate model based on the training sample to obtain a trained base calling error rate model.

In step (31) described above, the standard sample refers to a nucleic acid sample with a known base sequence. In the embodiments of the present application, the selection of the standard sample is not strictly required, and the standard sample may be a human reference gene sequence or a reference gene sequence of other species, as long as the sequence is known. In some embodiments, a sample with a larger genome is selected as the standard sample, and samples containing a plurality of different species may also be used together as the standard sample. By utilizing training data derived from samples with larger genomes or from genomes of multiple species, the training data can encompass a broader range of features, particularly the contextual sequence features required in the embodiments of the present application. This, in turn, enhances the generalization of the basecall model.

The sequence of the standard sample can be directly obtained through the information provided by the standard sample, or determined by sequencing the standard sample and then aligning the obtained sequence with a reference genome. Illustratively, on a selected sequencing platform, the standard sample is sequenced to obtain the corresponding base sequence. The obtained base sequence is then aligned with the reference genome of the standard sample to determine the known sequence.

In step (32) described above, the standard sample is subjected to a base extension reaction to acquire the fourth sequencing information of the sequencing cycle. In the embodiments of the present application, the fourth sequencing information at least includes the base calling probability scores of four base types. Optionally, the fourth sequencing information further includes the correct/incorrect classification information of the initial base calling result, such as a five-class classification. For the fourth sequencing information, reference may be made to the above description and the fourth sequencing information is not described further here.

In steps (33) and (34) described above, a training sample is constructed by using the fourth sequencing information of each sequencing cycle as a feature value and using the base calling error rate corresponding to the sequencing cycle as a target value. The pre-constructed base calling error rate model is trained based on the training sample to acquire the mapping relationship between the fourth sequencing information of the sequencing cycle and the base calling error rate, thereby obtaining the trained base calling error rate model.

It should be understood that when training the base calling error rate model, the base calling probability score of each of the four base types is used as an individual feature, that is, four features are separately inputted into a pre-constructed machine learning model/deep learning model for training. When the fourth sequencing information includes correct/incorrect classification information of the five-class classification, each of the five categories is used as an individual feature, that is, five features are separately inputted into the pre-constructed machine learning model/deep learning model for training. Combined with the base calling probability scores of the four base types, in this case, at least nine features are separately inputted into the pre-constructed machine learning model/deep learning model for training.

In step S1332 described above, the base calling error rate of the sequencing cycle to be processed can be determined based on the third mapping relationship and the fourth sequencing information of the sequencing cycle to be processed. In one embodiment, in step S1332, determining the base calling error rate of the sequencing cycle to be processed based on the third mapping relationship and the fourth sequencing information of the sequencing cycle to be processed includes:

acquiring the fourth sequencing information of the sequencing cycle to be processed; and
determining the base calling error rate of the sequencing cycle to be processed based on the fourth sequencing information and the third mapping relationship.

In some embodiments, the method further includes converting the base calling error rate into a Q value.

In some embodiments, the conversion of the base calling error rate into a Q value can be performed using the following formula: Q = -10 × log10 (P), where Q represents the quality score and P represents the base calling error probability.

The method for base calling provided in the embodiments of the present application can incorporate the utilized models as a supplementary module into the existing base calling algorithm software of the sequencing platform. During the sequencing process, the original algorithm software is first used to perform normal base calling of sequencing at predefined time points. Subsequently, the method employed in the embodiments of the present application is applied to re-identify or correct the base calling of the target sequencing cycle. Certainly, since this method requires contextual information, the sequencing platform's original base calling algorithm software, when performing base sequencing and identification in cycle N, may not be able to utilize the downstream sequence to identify the base of the target sequencing cycle. Instead, it need to wait until the base calling for a predetermined number of downstream cycles after cycle N is completed before re-identifying and correcting the base of cycle N using the downstream sequence information.

The method for base calling provided in this embodiment is illustrated below by specific examples.

The method for base calling provided in this embodiment includes correcting base calling results based on *a* consecutive sequencing cycles, including:

(1) Constructing a whole-genome (WGS) library using the human genome standard sample HG001, performing standard single-chip dual-end 150-cycle sequencing on a sequencing platform, and simultaneously performing base calling using the existing associated base calling software. This software can locate the position of the DNA template clusters to be tested on the raw sequencing images, and then identify the base corresponding to each cycle of sequencing for each template. The sequencing results were then aligned with the human genome reference genome HG19. It should be noted that other genomic datasets may also be combined, such as datasets from other species' genomes, to improve the diversity of the data.

(2) Using sequencing data from 1200 different fields of view corresponding to 10 experimental groups as the total dataset. In this total dataset, base coverage corresponding to polymorphic sites had been excluded to avoid the impact of inaccurate reference genomes on model training (in this embodiment, suspected polymorphic site regions can be removed by purchasing standard reference materials or using reference standard gene library information). Both the training set and the test set of this embodiment were sampled from the total dataset. Specifically:

(21) Training set 1 was extracted from the total dataset.

The contextual sequence and correct and incorrect information of sequencing were extracted for each base: the adjacent contextual 30 sequencing bases corresponding to each base were extracted as the contextual sequence, that is, the upstream sequence and the downstream sequence were each a string of 30 bases. In the case that the contextual sequence was less than 30 bases, the total length is filled with N to 30 bases in the direction where the contextual sequence is away from the base to be analyzed. The correct and incorrect information of the base was set as a five-class classification, namely: "correctly identified", "A base misidentified (should be A)", "C base misidentified (should be C)", "G base misidentified (should be G)", and "T or U base misidentified (should be T or U)".

(22) Using the data from training set 1, a classification model was constructed based on a convolutional neural network to predict the impact of the context on the current base calling, where the upstream sequence, downstream sequence, and the actual base type read (i.e., the base type of the target sequencing cycle) served as the input feature, and the correct and incorrect information of the base served as the target value. This model was referred to herein as a "model for correct and incorrect base calling classification". The output of the model for correct and incorrect base calling classification consists of five probability scores corresponding to the five-class classification.

The feature value of the "contextual sequence" may be represented using either of the two ways in the example described above. Optionally, the contextual information may be inputted by treating each base as an independent feature for training, or by treating the entire context or the upstream or downstream sequence as a string (i.e., one feature) for training. When the entire context is used as one string input feature, the feature quantity of the model can be significantly reduced, thereby decreasing the model complexity.

(23) Training set 2 was extracted (training set 2 did not intersect with training set 1).

The corrected brightness from four fluorescence channels corresponding to each base, and the corrected fluorescence brightness from the four fluorescence channels corresponding to the preceding and succeeding bases were extracted. Additionally, the base quality score corresponding to the current base was extracted. The base from the aligned reference genome corresponding to each base was extracted as the "ground truth" (standard base).

(24) Using the data from training set 2, a machine learning model was constructed using Light GBM, where the four-channel brightness corresponding to the current base, the preceding base, and the succeeding base described above, the base quality score corresponding to the current base, and five probability scores obtained for each base using the model for correct and incorrect base calling classification established in aforementioned step (22) were served together as the input feature, and the "ground truth" in the aligned reference genome corresponding to each base served as the target value. This model was referred to herein simply as a "basecall model".

It should be noted that when constructing the basecall model, the brightness information corresponding to specific preceding and succeeding sequencing cycles may be selected as feature inputs. Moreover, the contextual range of the brightness feature required to be inputted and the contextual range of the sequence required to be inputted do not need to be identical. Therefore, a broader contextual sequence range may be selected as input, while simultaneously selecting a smaller range of brightness information or even brightness information excluding contextual information as input.

(25) A test set was extracted from the total dataset. This test set had no intersection with both training set 1 and training set 2. The total error rate decreased by about 49% on the test set.

(3) Integrating the "model for correct and incorrect base calling classification" and the "basecall model" into base calling software associated with the sequencing platform described above to generate new base calling software.

After generating the new base calling software, testing was conducted for the new base calling software. Specifically, a whole-exome sequencing (WES) library was constructed using the human genome standard sample HG002. Standard single-chip dual-end 150-cycle sequencing was performed on the GenoLab M platform using the associated new base calling software. After sequencing was completed, the raw sequencing images from this experiment were reanalyzed using the previous version base calling software to obtain sequencing results of the previous version of base calling software.

(4) Performing further bioinformatics analysis on the sequencing results generated by the previous and new versions of base calling software described above. Only the high-confidence regions of the HG002 library genome were selected (analyzing based on the sequencing data from multiple platforms available online, and filtering out regions where the data is greater than the preset probability and polymorphic sites and other difficult-to-process areas were not contained) as the analysis subjects. For the "analysis subject" described above, it was considered that the sequencing results covering the corresponding regions should be identical to the bases in the corresponding regions of the human reference genome HG19; otherwise, sequencing errors were considered to have occurred.

The statistical analysis of the bioinformatics characteristics of the sequencing results corresponding to the base calling software before and after improvement was performed, respectively. In one aspect, the sequencing error rate corresponding to the base calling software of this embodiment was reduced by 45% compared to that of the base calling software before improvement (the original base calling software of the sequencing platform). In another aspect, the mutation frequency at each site in the "analysis subject" regions described above was statistically analyzed. If the error rate of the covered base at a certain site exceeded 2% (the positive and negative strands were separately analyzed), the site was considered as a "false mutation" site. Compared to the base calling software before improvement, the number of the "false mutation" sites of the base calling software of this embodiment was reduced by 79%. Therefore, after using the base calling software of this embodiment, not only the overall error rate was decreased, but the number of sites prone to high-frequency errors was also significantly decreased.

FIG. 2 is a base calling apparatus according to an embodiment of the present disclosure, and the apparatus is configured to implement the steps of the method for base calling described above. The description of the technical features and advantages of the embodiments of the method for base calling is also applicable to the apparatus, and the details are not repeated here. It can be understood that additional technical features of the method for base calling in any of the embodiments described above, including sub-steps, additional steps, and optional, alternative or better settings or processing can be implemented by allowing the apparatus or modules of the apparatus to further include units/modules or subunits/submodules.

As shown in FIG. 2, the base calling apparatus includes:

> a first mapping relationship acquisition module 210, configured to acquire a first mapping relationship between correct/incorrect classification information of an initial base calling result of a sequencing cycle and first sequencing information of the initial base calling result of a sequencing cycle, where the first sequencing information includes first initial base calling information based on a target sequencing cycle, the first initial base calling information includes initial base calling information of *a* consecutive sequencing cycles, and the *a* consecutive sequencing cycles at least include the target sequencing cycle;
> a correct/incorrect classification information determination module 220, configured to determine the correct/incorrect classification information of the initial base calling result of a sequencing cycle to be processed based on the first mapping relationship and the first sequencing information of the sequencing cycle to be processed.

In some embodiments of the first mapping relationship acquisition module 210, the first initial base calling information includes initial base calling information of *a* consecutive sequencing cycles, and the *a* consecutive sequencing cycles at least include the designated sequencing cycle.

In some embodiments of the first mapping relationship acquisition module 210, the *a* consecutive sequencing cycles include the target sequencing cycle and at least one of the following two scenarios:

$n_1$ sequencing cycles where base extension reactions are performed prior to the target sequencing cycle, and
$m_1$ sequencing cycles where base extension reactions are performed after the target sequencing cycle, where $n_1$ and $m_1$ are each independently an integer greater than or equal to 0, and $m_1$ and $n_1$ are not simultaneously 0.

In some embodiments of the first mapping relationship acquisition module 210, the first initial base calling information includes initial base calling information sequence of $a$ consecutive sequencing cycles.

In some embodiments of the first mapping relationship acquisition module 210, the first initial base calling information further includes initial base calling information of the target sequencing cycle.

In some embodiments of the first mapping relationship acquisition module 210, the first initial base calling information includes initial base calling information of each sequencing cycle of the $n_1$ sequencing cycles and/or the $m_1$ sequencing cycles, and initial base calling information of the target sequencing cycle.

In some embodiments of the first mapping relationship acquisition module 210, the first initial base calling information includes initial base calling information of each sequencing cycle of the $a$ consecutive sequencing cycles.

In some embodiments of the first mapping relationship acquisition module 210, the first initial base calling information includes an initial base calling information sequence formed by at least one of the $n_1$ sequencing cycles and/or the $m_1$ sequencing cycles and the target sequencing cycle, and initial base calling information of the target sequencing cycle.

In some embodiments of the first mapping relationship acquisition module 210, the first initial base calling information is a optical signal intensity generated during a base extension reaction.

In some embodiments of the first mapping relationship acquisition module 210, the optical signal intensity includes optical signal intensities of a plurality of optical signal channels, where the plurality of optical signals respectively correspond to a plurality of base types involved in the base extension reaction.

In some embodiments of the first mapping relationship acquisition module 210, the optical signal intensity is the highest optical signal intensity among the plurality of optical signal channels.

In some embodiments of the first mapping relationship acquisition module 210, the optical signal intensity is a fluorescence signal intensity.

In some embodiments of the first mapping relationship acquisition module 210, the fluorescence signal intensity includes any one of an initial fluorescence signal intensity and a corrected fluorescence signal intensity.

In some embodiments of the first mapping relationship acquisition module 210, the correction includes at least one of a background correction, a crosstalk correction, a reaction asynchrony correction, and a normalization process.

In some embodiments, the correct/incorrect classification information may be correct and incorrect categories, including a correct category and an incorrect category. In some embodiments, the correct/incorrect category may be a binary classification, i.e., a correct category and an incorrect category. In one implementation, if the initial base calling result of the base extension in the sequencing cycle matches the real base, the initial base calling of the sequencing cycle is considered correct and is classified under the correct category in the correct/incorrect classification information. Illustratively, if the initial base calling result of the base extension in the sequencing cycle is A, and the reagent base type is also A, the initial base calling of the sequencing cycle is considered correct. In this case, it is classified under the correct category in the correct/incorrect classification information. In another implementation, if the initial base calling result of the base extension of the sequencing cycle does not match the real base, the initial base calling of the sequencing cycle is considered incorrect and is classified as the incorrect category in the correct/incorrect classification information. Illustratively, if the initial base calling result of the base extension in the sequencing cycle is A and the real base is T, the initial base calling of the sequencing cycle is considered incorrect. In this case, it is classified under the incorrect category in the correct/incorrect classification information.

In some embodiments, the incorrect category may be further classified based on different types of errors, such that the correct/incorrect category is a multi-class classification with more than two categories. In one embodiment, the correct/incorrect category is a five-class classification, and the five-class classification includes the following five categories:

a correct category of the initial base calling result;
an incorrect category of A base;
an incorrect category of T or U base;
an incorrect category of G base; and
an incorrect category of C base.

The classification criterion for the incorrect category of A base is as follows: the corresponding real base of the sequencing cycle is A, but the initial base calling result of the base extension in the sequencing cycle is not A, and in this case, the correct/incorrect category of the initial base calling of the sequencing cycle is considered to fall into: the incorrect category of A base. In some embodiments, the incorrect category of A base may be further classified based on the initial base calling result of the base extension in the sequencing cycle, for example, a category where A base is misidentified as T, a category where A base is misidentified as G, and a category where A base is misidentified as C.

Similarly, the classification criterion for the incorrect category of T or U base is as follows: the corresponding real base of the sequencing cycle is T, but the initial base calling result of the base extension in the sequencing cycle is not T, and in this case, the correct/incorrect category of the initial base calling of the sequencing cycle is considered to fall into: the incorrect category of T or U base. In some embodiments, the incorrect category of T or U base may be further classified based on the initial base calling result of the base extension in the sequencing cycle, for example, a category where T or U base is misidentified as A, a category where T or U base is misidentified as G, and a category where T or U base is misidentified as C.

The classification criterion for the incorrect category of G base is as follows: the corresponding real base of the sequencing cycle is G, but the initial base calling result of the base extension in the sequencing cycle is not G, and in this case, the correct/incorrect category of the initial base calling of the sequencing cycle is considered to fall into: the incorrect category of G base. In some embodiments, the incorrect category of G base may be further classified based on the initial base calling result of the base extension in the sequencing cycle, for example, a category where G base is misidentified as T, a category where G base is misidentified as A, and a category where G base is misidentified as C.

The classification criterion for the incorrect category of C base is as follows: the corresponding real base of the sequencing cycle is C, but the initial base calling result of the base extension in the sequencing cycle is not C, and in this case, the correct/incorrect category of the initial base calling of the sequencing cycle is considered to fall into: the incorrect category of C base. In some embodiments, the incorrect category of C base may be further classified based on the initial base calling result of the base extension in the sequencing cycle, for example, a category where C base is misidentified as A, a category where C base is misidentified as G, and a category where C base is misidentified as T.

In some embodiments of the first mapping relationship acquisition module 210, the first mapping relationship is obtained by training a pre-constructed Correct/Incorrect Base Calling Classification Model.

In some embodiments, the training of the Correct/Incorrect Base Calling Classification Model includes: acquiring a standard sample and a sequence of the standard sample;

subjecting the standard sample to a base extension reaction, and determining second sequencing information of the target sequencing cycle and the initial base calling result of base extension in the target sequencing cycle, where the second sequencing information at least includes the first sequencing information;
determining correct and incorrect categories of the initial base calling of the target sequencing cycle based on the initial base calling result of the base extension in the target sequencing cycle and a standard base;
constructing a training sample by using the second sequencing information as a feature value and using the correct/incorrect category of the initial base calling sequence as a target value; and training the pre-constructed Correct/Incorrect Base Calling Classification Model based on the training sample to obtain a trained Correct/Incorrect Base Calling Classification Model.

In some embodiments, the correct/incorrect classification information determination module 220 includes:

a first sequencing information submodule, configured to acquire the first sequencing information of a sequencing cycle to be processed; and
a correct/incorrect classification information determination submodule, configured to determine the correct/incorrect classification information of the initial base calling result of the sequencing cycle to be processed based on the first sequencing information and the first mapping relationship.

In some embodiments, the base calling apparatus further includes:
a base calling result determination module 230, configured to determine the base calling result of the sequencing cycle to be processed based on the correct/incorrect classification information of the initial base calling result.

In some embodiments, the base calling result determination module 230 includes:

a second mapping relationship acquisition module, configured to acquire a second mapping relationship between the standard base of the sequencing cycle and third sequencing information, where the third sequencing information includes second initial base calling information based on a target sequencing cycle and the correct/incorrect classification information of the initial base calling result; and
a base calling result determination submodule, configured to determine the base calling result of the sequencing cycle to be processed based on the second mapping relationship and the third sequencing information of the sequencing cycle to be processed.

In some embodiments, the base calling result determination submodule is specifically configured to: acquire the third sequencing information of the sequencing cycle to be processed; and determine probabilities of four base types of the sequencing cycle to be processed based on the third sequencing information and the second mapping relationship, and take the base type with the highest probability as the base calling result of the sequencing cycle to be processed.

In some embodiments, the second initial base calling information includes the initial base calling information of the target sequencing cycle;

the second initial base calling information is the optical signal intensity generated during a base extension reaction in the target sequencing cycle.

In some embodiments, the optical signal intensity includes optical signal intensities of a plurality of optical signal channels, where the plurality of optical signals respectively correspond to a plurality of base types involved in the base extension reaction.

In some embodiments, the optical signal intensity is the highest optical signal intensity among the plurality of optical signal channels.

In some embodiments, the optical signal intensity is a fluorescence signal intensity.

In some embodiments, the fluorescence signal intensity includes any one of an initial fluorescence signal intensity and a corrected fluorescence signal intensity.

In some embodiments, the correction includes at least one of a background correction, a crosstalk correction, a reaction asynchrony correction, and a normalization process.

In some embodiments, the third sequencing information further includes at least one of the following information:

an E value from the target sequencing cycle;
a sequence determined based on an E value of each sequencing cycle in b consecutive sequencing cycles; optical signal intensities generated in each sequencing cycle in b consecutive sequencing cycles,

The E value is used to evaluate the quality of sequencing data, and is generally indicative of the accuracy of the sequencing data. Specifically, the E value is the ratio of the maximum value of optical signal intensities of the four base types in optical detection channels to the sum of the optical signal intensities of the four base types.

In some embodiments, the b consecutive sequencing cycles are selected from at least one of the following:

the target sequencing cycle and $n_2$ sequencing cycles where base extension reactions are performed prior to the target sequencing cycle; or
the target sequencing cycle and $m_2$ sequencing cycles where base extension reactions are performed after the target sequencing cycle; or
the target sequencing cycle, $n_2$ sequencing cycles where sequencing is completed prior to the target sequencing cycle, and $m_2$ sequencing cycles where sequencing is completed after the target sequencing

cycle,
where $n_2$ and $m_2$ are each independently an integer greater than or equal to 0, and $m_2$ and $n_2$ are not simultaneously 0.

In some embodiments, the base calling apparatus further includes:
a base calling error rate determination module 240, configured to determine a base calling error rate of the sequencing cycle to be processed based on the base calling result.

In some embodiments, the base calling error rate determination module includes:

a third mapping relationship acquisition module, configured to acquire a third mapping relationship between fourth sequencing information of the sequencing cycle and the base calling error rate, where the fourth sequencing information includes base calling probability scores of four base types; and
a base calling error rate determination submodule, configured to determine the base calling error rate of the sequencing cycle to be processed based on the third mapping relationship and the fourth sequencing information of the sequencing cycle to be processed.

In some embodiments, the fourth sequencing information further includes the correct/incorrect classification information of the initial base calling result, and the correct/incorrect category in the correct/incorrect classification information includes the following five categories:

a correct category of the initial base calling result;
an incorrect category of A base;
an incorrect category of T or U base;
an incorrect category of G base; and
an incorrect category of C base.

The base calling apparatus provided in the embodiments of the present disclosure can perform the method for base calling according to any embodiment of the present disclosure, and has corresponding modules for performing the method.

It should be understood that the features involved in the method for base calling can be used in the base calling apparatus to interpret the base calling apparatus and the modules to implement the steps of the method for base calling.

FIG. 3 is a schematic structural diagram of an electronic device according to an embodiment of the present disclosure. The electronic device 10 is intended to represent various forms of digital computers, such as a laptop, a desktop, a workstation, a personal digital assistant, a server, a blade server, a mainframe, and other appropriate computers. The electronic device may also represent various forms of mobile apparatuses, such as a personal digital assistant, a cellular phone, a smart phone, a wearable device (e.g., a helmet, glasses, a watch, etc.), and other similar computing apparatuses. The components shown herein, their connections and relationships, and their functions are merely exemplary and are not intended to limit the implementations of the present disclosure described and/or claimed herein.

As shown in FIG. 3, the electronic device 10 includes at least one processor 11 and a memory in communication connection to the at least one processor 11, such as a read-only memory (ROM) 12, or a random access memory (RAM) 13. The memory stores a computer program that can be run by the at least one processor. The processor 11 can perform various appropriate actions and processes based on the computer program stored in the read-only memory (ROM) 12 or the computer program loaded from a storage unit 18 into the random access memory (RAM) 13. In the RAM 13, various programs and data necessary for the operation of the electronic device 10 may also be stored. The processor 11, the ROM 12, and the RAM 13 are connected to each other via a bus 14. An input/output (I/O) interface 15 is also connected to the bus 14.

A plurality of components in the electronic device 10 are connected to the I/O interface 15, including: an input unit 16, such as a keyboard or a mouse; an output unit 17, such as various types of displays or speakers; a storage unit 18, such as a magnetic disk or an optical disk; and a communication unit 19, such as a network card, a modem, or a wireless communication transceiver. The communication unit 19 allows the electronic device 10 to exchange information/data with other devices through a computer network such as the Internet and/or various telecommunication networks.

The processor 11 may be various general-purpose and/or dedicated processing components having processing and computing capabilities. Some examples of the processor 11 include, but are not limited to, a central processing unit (CPU), a graphics processing unit (GPU), various dedicated artificial intelligence (AI) computing chips, various processors running machine learning model algorithms, a digital signal processor (DSP), and any suitable processor, controller, or microcontroller. The processor 11 performs the various methods and processes described above, such as the method for base calling.

In some embodiments, the method for base calling may be implemented as a computer program, which is tangibly embodied in a computer-readable storage medium, such as the storage unit 18. In some embodiments, part or all of the computer program can be loaded and/or installed onto the electronic device 10 via the ROM 12 and/or the communication unit 19. When the computer program is loaded into the RAM 13 and run by the processor 11, one or more steps of the method for base calling described above can be performed. Alternatively, in other embodiments, the processor 11 may be configured to perform the method for base calling by any other suitable means (e.g., by means of firmware).

The various embodiments of the system and techniques described above herein may be implemented in a digital electronic circuit system, an integrated circuit system, a field programmable gate array (FPGA), an application specific integrated circuit (ASIC), an application specific standard products (ASSP), a system on chip (SOC), a complex programmable logic device (CPLD), computer hardware, firmware, software, and/or combinations thereof. These various embodiments may include: implementation in one or more computer programs, which can be run and/or interpreted on a programmable system including at least one programmable processor. The programmable processor may be of special or general purpose and capable of receiving data and instructions from, as well as transmitting data and instructions to, a storage system, at least one input apparatus, and at least one output apparatus.

The computer programs for implementing the method for base calling of the present disclosure may be written in any combination of one or more programming languages. The computer programs may be provided to the processor of a general-purpose computer, a special-purpose computer, or other programmable data processing apparatuses, such that the computer programs, when run by the processor, enable the implementation of the functions/operations specified in the flowcharts and/or block diagrams. The computer programs can be run entirely on the machine, partially on the machine, as a stand-alone software package partially on the machine and partially on a remote machine, or entirely on a remote machine or server.

The embodiments of the present disclosure also provide a computer-readable storage medium. The computer-readable storage medium stores one or more computer instructions, and the one or more computer instructions, when executed by a processor, cause the processor to perform a method for base calling. The method includes:

acquiring a first mapping relationship between correct/incorrect classification information of an initial base calling result of a sequencing cycle and first sequencing information of the initial base calling result of a sequencing cycle, where the first sequencing information includes first initial base calling information based on a target sequencing cycle, the first initial base calling information includes initial base calling information of *a* consecutive sequencing cycles, and the *a* consecutive sequencing cycles at least includes the target sequencing cycle; and

determining the correct/incorrect classification information of the initial base calling result of a sequencing cycle to be processed based on the first mapping relationship and the first sequencing information of the sequencing cycle to be processed.

In the context of the present disclosure, the computer-readable storage medium may be a tangible medium, which may contain or store computer programs for use by, or in conjunction with, an instruction execution system, an apparatus, or a device. The computer-readable storage medium may include, but is not limited to, an electronic, magnetic, optical, electromagnetic, infrared, or semiconductor system, apparatus or device, or any suitable combination thereof. Alternatively, the computer-readable storage medium may be a machine-readable signal medium. More specific examples of a machine-readable storage medium would include an electrical connection based on one or more wires, a portable computer disk, a hard disk, a RAM, a ROM, an erasable programmable read-only memory (EPROM or flash memory), an optical fiber, a portable compact disc read-only memory (CD-ROM), an optical storage device, a magnetic storage device, or any suitable combination thereof. To provide user interaction, the system and techniques described herein may be implemented on an electronic device. The electronic device includes: a display apparatus (e.g., a cathode ray tube (CRT) or a liquid crystal display (LCD) monitor) for displaying information to a user; and a keyboard and a pointing device (e.g., a mouse or a trackball), through which a user may provide input to the electronic device. Other types of apparatuses may further be used to provide user interaction; for example, feedback provided to the user may be any form of sensory feedback (e.g., visual feedback, auditory feedback, or tactile feedback); and input from the user may be received in any form (including acoustic, speech, or tactile input).

The system and techniques described herein may be implemented in a computing system that includes a backend component (e.g., as a data server), or a computing system that includes a middleware component (e.g., an application server), or a computing system that includes a frontend component (e.g., a user computer having a graphical user interface or a web browser, through which a user can interact with the embodiment of the system and techniques described herein), or a computing system that includes any combination of such backend, middleware, or frontend components. The components of the system can be interconnected via any form or medium of digital data communication (e.g., communication networks). Examples of the communication networks include: local area networks (LAN), wide area networks (WAN), blockchain networks, and the Internet.

The computing system may include a client and a server. The client and the server are generally remote from each other and typically interact through a communication network. The client-server relationship is established by running computer programs on corresponding computers that operate in a client-server relationship to each other. The server may be a cloud server, also known as a cloud computing server or a cloud host, which is a type of host product in a cloud computing service system, designed to address the defects of traditional physical hosts and VPS services, such as high management difficulty and weak business scalability.

It should be understood that the various forms of processes shown above may be reordered, augmented, or reduced in steps. For example, various steps described in the present disclosure may be performed in parallel, sequentially, or in different orders, which is not limited herein as long as the desired results of the technical solutions of the present disclosure can be achieved.

The above specific embodiments do not limit the protection scope of the present disclosure. It should be understood by those skilled in the art that various modifications, combinations, sub-combinations, and substitutions may be made according to design requirements and other factors. Any modification, equivalent replacement, or improvement made within the spirit and principle of the present disclosure should be included in the protection scope of the present disclosure.

**Claims**

1. A method for base calling, comprising:

   acquiring a first mapping relationship between correct/incorrect classification information of an initial base calling result of a sequencing cycle and first sequencing information of the initial base calling result of a sequencing cycle, wherein the first sequencing information comprises first initial base calling information based on a target sequencing cycle, the first initial base calling information comprises initial base calling information of $a$ consecutive sequencing cycles, and the $a$ consecutive sequencing cycles at least comprise the target sequencing cycle; and determining the correct/incorrect classification information of the initial base calling result of a sequencing cycle to be processed based on the first mapping relationship and the first sequencing information of the sequencing cycle to be processed.

2. The method according to claim 1, wherein determining the correct/incorrect classification information of the initial base calling result of the sequencing cycle to be processed based on the first mapping relationship and the first sequencing information of the sequencing cycle to be processed comprises:

   acquiring the first sequencing information of the sequencing cycle to be processed; and determining the correct/incorrect classification information of the initial base calling result of the sequencing cycle to be processed based on the first sequencing information and the first mapping relationship.

**3.** The method according to claim 1 or 2, wherein the first initial base calling information comprises the initial base calling information of the *a* consecutive sequencing cycles, and the *a* consecutive sequencing cycles at least comprise the designated sequencing cycle;

the *a* consecutive sequencing cycles comprise the target sequencing cycle and at least one of the following two scenarios:

$n_1$ sequencing cycles wherein base extension reactions are performed prior to the target sequencing cycle,

$m_1$ sequencing cycles wherein base extension reactions are performed after the target sequencing cycle,

wherein $n_1$ and $m_1$ are each independently an integer greater than or equal to 0, and $m_1$ and $n_1$ are not simultaneously 0.

**4.** The method according to claim 3, wherein the first initial base calling information comprises an initial base calling information sequence of the *a* consecutive sequencing cycles.

wherein optionally the first initial base calling information further comprises initial base calling information of the target sequencing cycle;

or the first initial base calling information comprises initial base calling information of each sequencing cycle of the $n_1$ sequencing cycles and/or the $m_1$ sequencing cycles, and initial base calling information of the target sequencing cycle;

or the first initial base calling information comprises initial base calling information of each sequencing cycle of the *a* consecutive sequencing cycles;

or the first initial base calling information comprises an initial base calling information sequence formed by at least one of the $n_1$ sequencing cycles and/or the $m_1$ sequencing cycles and the target sequencing cycle, and initial base calling information of the target sequencing cycle.

**5.** The method according to any one of claims 3 to 4, wherein the first initial base calling information is a optical signal intensity generated during a base extension reaction;

wherein optionally the optical signal intensity comprises optical signal intensities of a plurality of optical signal channels, wherein the plurality of optical signals respectively correspond to a plurality of base types involved in the base extension reaction;

wherein optionally the optical signal intensity is the highest optical signal intensity among the plurality of optical signal channels;

wherein optionally the optical signal intensity is a fluorescence signal intensity;

wherein optionally the fluorescence signal intensity comprises any one of an initial fluorescence signal intensity and a corrected fluorescence signal intensity;

wherein optionally the correction comprises at least one of a background correction, a crosstalk correction, a reaction asynchrony correction, and a normalization process.

**6.** The method according to any one of claims 1 to 5, wherein the first mapping relationship is obtained by training a pre-constructed Correct/Incorrect Base Calling Classification Model;

wherein optionally the training of the Correct/Incorrect Base Calling Classification Model comprises:

acquiring a standard sample and a sequence of the standard sample;

subjecting the standard sample to a base extension reaction, and determining second sequencing information of the target sequencing cycle and the initial base calling result of base extension in the target sequencing cycle, wherein the second sequencing information at least comprises the first sequencing information;

determining correct and incorrect categories of the initial base calling of the target sequencing cycle based on the initial base calling result of the base extension in the target sequencing cycle and a standard base;

constructing a training sample by using the second sequencing information as a feature value and using the correct/incorrect category of the initial base calling sequence as a target value; and

training the pre-constructed Correct/Incorrect Base Calling Classification Model based on the training sample to obtain a trained Correct/Incorrect Base Calling Classification Model;

wherein optionally the correct/incorrect category may be a binary classification or a multiclass classification with more than two categories;

wherein optionally the correct/incorrect category comprises the following two categories: the correct category of the initial base calling result and the incorrect category of the initial base calling result;

wherein optionally the correct/incorrect category comprises the following five categories:

a correct category of the initial base calling result;

an incorrect category of A base;

an incorrect category of T or U base;
an incorrect category of G base; and
an incorrect category of C base.

7. The method according to any one of claims 1 to 6, the method for base calling further comprises:
determining the base calling result of the sequencing cycle to be processed based on the correct/incorrect classification information of the initial base calling result.

8. The method according to claim 7, wherein determining the base calling result of the sequencing cycle to be processed based on the correct/incorrect classification information of the base calling comprises:

acquiring a second mapping relationship between the standard base of the sequencing cycle and third sequencing information, wherein the third sequencing information comprises second initial base calling information based on a target sequencing cycle and the correct/incorrect classification information of the initial base calling result; and
determining the base calling result of the sequencing cycle to be processed based on the second mapping relationship and the third sequencing information of the sequencing cycle to be processed.

9. The method according to claim 8, wherein determining the initial base calling result of the sequencing cycle to be processed based on the second mapping relationship and the third sequencing information of the sequencing cycle to be processed comprises:

acquiring the third sequencing information of the sequencing cycle to be processed; and
determining probabilities of four base types of the sequencing cycle to be processed based on the third sequencing information and the second mapping relationship, and taking the base type with the highest probability as the base calling result of the sequencing cycle to be processed.

10. The method according to claim 8, wherein the second initial base calling information comprises initial base calling information of the target sequencing cycle;

the second initial base calling information is the optical signal intensity generated during a base extension reaction in the target sequencing cycle;
wherein optionally the optical signal intensity comprises optical signal intensities of a plurality of optical signal channels, wherein the plurality of optical signals respectively correspond to a plurality of base types involved in the base extension reaction;
wherein optionally the optical signal intensity is the highest optical signal intensity among the plurality of optical signal channels;
wherein optionally the optical signal intensity is a fluorescence signal intensity;
wherein optionally the fluorescence signal intensity comprises any one of an initial fluorescence signal intensity and a corrected fluorescence signal intensity;
wherein optionally the correction comprises at least one of a background correction, a crosstalk correction, a reaction asynchrony correction, and a normalization process.

11. The method according to any one of claims 7 to 10, wherein the third sequencing information further comprises at least one of the following information:

an E value from the target sequencing cycle;
a sequence determined based on an E value of each sequencing cycle in b consecutive sequencing cycles;
optical signal intensities generated in each sequencing cycle in b consecutive sequencing cycles,
wherein the E value is the ratio of the maximum value of optical signal intensities of four base types in optical detection channels to the sum of the optical signal intensities of the four base types;
wherein optionally the b consecutive sequencing cycles are selected from at least one of the following:

the target sequencing cycle and $n_2$ sequencing cycles wherein base extension reactions are performed prior to the target sequencing cycle; or
the target sequencing cycle and $m_2$ sequencing cycles wherein base extension reactions are performed after the target sequencing cycle; or
the target sequencing cycle, $n_2$ sequencing cycles wherein sequencing is completed prior to the target sequencing cycle, and $m_2$ sequencing cycles wherein sequencing is completed after the target sequencing cycle,
wherein $n_2$ and $m_2$ are each independently an integer greater than or equal to 0, and $m_2$ and $n_2$ are not simultaneously 0.

12. The method according to any one of claims 7 to 11, wherein the method for base calling further comprises:

determining a base calling error rate of the sequencing cycle to be processed based on the base calling result;

wherein optionally determining the base calling error rate of the sequencing cycle to be processed based on the base calling result comprises:

acquiring a third mapping relationship between fourth sequencing information of the sequencing cycle and the base calling error rate, wherein the fourth sequencing information comprises base calling probability scores of four base types; and

determining the base calling error rate of the sequencing cycle to be processed based on the third mapping relationship and the fourth sequencing information of the sequencing cycle to be processed;

wherein optionally the fourth sequencing information further comprises the correct/incorrect classification information of the initial base calling result, and the correct/incorrect category in the correct/incorrect classification information comprises the following five categories:

a correct category of the initial base calling result;
an incorrect category of A base;
an incorrect category of T or U base;
an incorrect category of G base; and
an incorrect category of C base.

13. A base calling apparatus, comprising:

a first mapping relationship acquisition module, configured to acquire a first mapping relationship between correct/incorrect classification information of an initial base calling result of a sequencing cycle and first sequencing information of the initial base calling result of a sequencing cycle, wherein the first sequencing information comprises first initial base calling information based on a target sequencing cycle, the first initial base calling information comprises initial base calling information of *a* consecutive sequencing cycles, and the *a* consecutive sequencing cycles at least comprise the target sequencing cycle;

a correct/incorrect classification information determination module, configured to determine the correct/incorrect classification information of the initial base calling result of a sequencing cycle to be processed based on the first mapping relationship and the first sequencing information of the sequencing cycle to be processed.

14. An electronic device, comprising:

at least one processor; and
a memory in communication connection to the at least one processor, wherein
the memory stores one or more computer programs run by the at least one processor, and the one or more computer programs, when run by the at least one processor, cause the at least one processor to perform the method for base calling,
the method for base calling, comprising:

acquiring a first mapping relationship between correct/incorrect classification information of an initial base calling result of a sequencing cycle and first sequencing information of the initial base calling result of a sequencing cycle, wherein the first sequencing information comprises first initial base calling information based on a target sequencing cycle, the first initial base calling information comprises initial base calling information of *a* consecutive sequencing cycles, and the *a* consecutive sequencing cycles at least comprise the target sequencing cycle; and

determining the correct/incorrect classification information of the initial base calling result of a sequencing cycle to be processed based on the first mapping relationship and the first sequencing information of the sequencing cycle to be processed.

15. A computer-readable storage medium, storing one or more computer instructions, wherein the one or more computer instructions, when executed by a processor, cause the processor to perform the method for base calling,
the method for base calling, comprising:

acquiring a first mapping relationship between correct/incorrect classification information of an initial base calling result of a sequencing cycle and first sequencing information of the initial base calling result of a sequencing cycle, wherein the first sequencing information comprises first initial base calling information based on a target sequencing cycle, the first initial base calling information comprises initial base calling information of *a* consecutive sequencing cycles, and the *a* consecutive sequencing cycles at least comprise the target sequencing cycle; and

determining the correct/incorrect classification information of the initial base calling result of a sequencing cycle to be processed based on the first mapping relationship and the first sequencing information of the sequencing cycle to be

**EP 4 645 317 A1**

processed.

Acquiring a mapping relationship between the standard base of a sequencing cycle and first sequencing information, where the first sequencing information includes first initial base calling information based on the designated sequencing cycle, as well as first initial base calling information based on a consecutive sequencing cycles, and the a consecutive sequencing cycles include the designated sequencing cycle

S110

Determining a base calling result of the sequencing cycle to be processed based on the mapping relationship and the first sequencing information of the sequencing cycle to be processed

S120

FIG. 1

210

220

First mapping relationship acquisition module

Correct and incorrect classification information determination module

FIG. 2

210

220

First mapping relationship acquisition module

Correct and incorrect classification information determination module

230

Base calling result determination module

FIG. 3

FIG. 4

FIG. 5

Europäisches
Patentamt
European
Patent Office
Office européen
des brevets

**EUROPEAN SEARCH REPORT**

Application Number

EP 25 17 3115

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/169824 A1 (KERMANI BAHRAM GHAFFARZADEH [US] ET AL) 18 June 2015 (2015-06-18) * figures 1-6 * * paragraph [0046] - paragraph [0090] * * paragraph [0159] - paragraph [0167] * * paragraph [0211] - paragraph [0216] * ----- | 1-15 | INV. G16B20/20 G16B30/20 G16B40/20 |
| A | AU 2021 224 592 A1 (ILLUMINA INC [US]) 8 September 2022 (2022-09-08) * paragraph [0059] - paragraph [0113]; figure 1 * ----- | 1-15 | |
| A | US 2020/302297 A1 (JAGANATHAN KISHORE [US] ET AL) 24 September 2020 (2020-09-24) * figures 1-5 * * paragraph [0168] - paragraph [0262] * ----- | 1-15 | |
| | | | TECHNICAL FIELDS SEARCHED (IPC) |
| | | | G16B |

The present search report has been drawn up for all claims

| Place of search | Date of completion of the search | Examiner |
|---|---|---|
| Munich | 19 September 2025 | Schechner-Resom, G |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

................................................................................
& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 25 17 3115

This annex lists the patent family members relating to the patent documents cited in  the above-mentioned European search report.
The members are as contained in the European Patent Office EDP file on
The European Patent Office is in no way liable for these particulars which are merely given for the purpose of information.

19-09-2025

| Patent document cited in search report | Publication date | Patent family member(s) | | Publication date |
|---|---|---|---|---|
| US 2015169824 A1 | 18-06-2015 | CN | 105980578 A | 28-09-2016 |
| | | EP | 3084002 A1 | 26-10-2016 |
| | | US | 2015169824 A1 | 18-06-2015 |
| | | WO | 2015095066 A1 | 25-06-2015 |
| AU 2021224592 A1 | 08-09-2022 | AU | 2021224592 A1 | 08-09-2022 |
| | | BR | 112022016409 A2 | 11-10-2022 |
| | | CA | 3168451 A1 | 26-08-2021 |
| | | CN | 115136245 A | 30-09-2022 |
| | | EP | 4107737 A1 | 28-12-2022 |
| | | IL | 295568 A | 01-10-2022 |
| | | JP | 2023515110 A | 12-04-2023 |
| | | KR | 20220144372 A | 26-10-2022 |
| | | US | 2021265018 A1 | 26-08-2021 |
| | | WO | 2021168014 A1 | 26-08-2021 |
| US 2020302297 A1 | 24-09-2020 | US | 2020302224 A1 | 24-09-2020 |
| | | US | 2020302225 A1 | 24-09-2020 |
| | | US | 2020302297 A1 | 24-09-2020 |
| | | US | 2020327377 A1 | 15-10-2020 |

EPO FORM P0459

For more details about this annex : see Official Journal of the European Patent Office, No. 12/82